# EUROPEAN PATENT APPLICATION

(11) **EP 4 725 440 A1**
(43) Date of publication of application: **15.04.2026**
(21) Application number: 25207515.5
(22) Date of filing: 08.10.2025
(51) Int. Cl.: A61B 34/20, A61B 17/56, A61B 34/30, A61B 34/32, A61B 34/00, A61B 17/17

(54) **SURGICAL ROBOT CONTROLLER WITH ATTRACTION VOLUME FOR HIP PROCEDURE**

(30) Priority: 08.10.2024 US 202418909473
(71) Applicant: Globus Medical, Inc, Audubon, PA 19403 (US)
(72) Inventor: CHAPPUIS, Olivier, 1095 LUTRY (CH); GEHRIGER, Daniel, 1004 LAUSANNE (CH); CAMERON, Hayden, PHILADELPHIA, 19129 (US); ANTAL, Marton, 1004 LAUSANNE (CH)
(74) Representative: Morabito, Sara

(57) **Abstract**

Aspects include a surgical robot for use in a total hip arthroplasty (THA) procedure, and related methods. In some cases, the surgical robot includes: a robot arm for holding a surgical instrument; and a controller coupled with the robot arm, the controller programmed to: detect a position of the surgical instrument in at least one attraction volume corresponding with a planned trajectory of the surgical instrument for the THA procedure, the at least one attraction volume defined in part by a point and an axis extending from the point; and while the surgical instrument is in the at least one attraction volume during the THA procedure, provide assistance to movement of the surgical instrument in a direction toward the at least one attraction volume.

## Description

### TECHNICAL FIELD

The invention relates generally to devices, systems, and methods for use in robot-assisted surgical procedures. More particularly, the invention relates to computer-assisted devices, systems, and methods for performing robot-assisted bone preparation, trialing, and implant placement during total hip arthroplasty (THA) surgical procedures. Various particular implementations use attraction volume-based approaches to guide a user in a THA surgical procedure.

### BACKGROUND OF THE INVENTION

Hip arthroplasty, or hip replacement, is a surgical procedure used to resurface and reconstruct a hip joint that has been damaged by disease or injury, e.g., by arthritis or a fracture. THA devices replace both the acetabulum and the femoral head that collectively comprise the hip joint. An acetabular implant is secured to the acetabulum, forming a replacement articulating surface which interfaces with the femoral implant secured to the end of the femur. The femoral implant is pivotably coupled to the acetabular implant, thereby reconstructing the hip joint. Exemplary acetabular implants are disclosed in, e.g., US Patent Application No. 17/024,876, filed September 18, 2020 (published as US 2022/0087823 A1), which is incorporated by reference as though fully set forth herein.

Robotic surgery systems including computer-assisted navigation have become a well-established technique in operating rooms, including their use in arthroplasty procedures. Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan. Camera tracking systems for computer assisted surgery navigation typically use a set of tracking cameras to track a pose of a reference element on the surgical instrument, which may be coupled to a surgical robot and may be positioned by a surgeon during surgery, relative to a patient reference element (or "dynamic reference base" (DRB)) affixed to the patient. A computer model of a real instrument is associated with a reference element, so that the computer model can be overlaid on registered images of patient's anatomy. The camera tracking system uses the relative poses of the reference elements to determine how the real instrument is posed relative to the patient and to determine how the computer model of the real instrument is to be correspondingly posed as overlaid on the medical images. The surgeon can thereby use real-time visual feedback of the relative poses to navigate the surgical instrument during a surgical procedure on the patient.

As noted above, a robotic system may be used for arthroplasty procedures. The robotic system (or, "robot" or "surgical robot") has a serial arm on which an end effector is mounted. The surgeon (or "user") may hold the end effector or any instruments coupled thereto, to perform surgical operations while watching in real time on a navigation system (e.g., on stand-alone display(s) or an Augmented Reality (AR) headset), and to receive various types of relevant feedback and information associated with a defined plan for and/or progress of the surgical procedure.

The serial arm can move through computer guided control to a suitable position for the surgery, e.g., pursuant to the surgeon's request, which may be provided via a foot pedal, touchscreen, AR interaction, etc. The passive robotic structure allows the surgeon to precisely perform each operation in the procedure.

Various workflows can be available for use with the system. Such workflows may incorporate preoperative scans or images of the patient (e.g., x-ray or Computerized Tomography (CT)). On the other hand, other workflows may be imageless, and may not require any pre-operative images. Some workflows may incorporate acquisition of intra-operative information about the patient anatomy. In one example, the surgeon may measure key parameters of the bone using a camera tracking system and an appropriate tracked instrument to capture points on patient anatomy. Later, this information, and other intra-operatively-acquired information may be used to plan the implant position and orientation with respect to patient anatomy, and to navigate the robot and surgical instruments during the surgical procedure.

In some workflows, the surgeon may rigidly attach a reference element to one or more bones, where the reference element includes fiducials which are detected by tracking cameras for computer assisted navigation. The reference elements allow tracking of bone position by the navigation system. The reference elements can be positioned on the bone and oriented such that they can be seen by the tracking cameras of the navigation system. Once positioned, the reference elements are attached with fixation structures (e.g., screw pins, "crocodile" jaws) on the bone (e.g., pelvis or femur). The reference elements' respective positions and orientations stay rigidly fixed with respect to the bone throughout the procedure.

Another process of various workflows is to register the patient in the tracking space of the navigation system. Patient registration can include matching the patient anatomy with a numeric representation of the corresponding bone, such as a three-dimensional (3D) model of the bone. The bone representation may be constructed from, e.g., a set of CT images (CT workflow), a set of fluoroscopy images, or based on a generic bone model (imageless workflow).

Although current surgical approaches offer sophisticated techniques in robotic and navigation-assisted surgeries, current approaches for bone preparation, trialing, and implant placement may have shortcomings, e.g., in THA procedures.

### BRIEF DESCRIPTION OF THE INVENTION

A first aspect of the disclosure provides a surgical robot for use in a total hip arthroplasty (THA) procedure, the robot including: a robot arm for holding a surgical instrument; and a controller coupled with the robot arm, the controller programmed to: detect a position of the surgical instrument in at least one attraction volume corresponding with a planned trajectory of the surgical instrument for the THA procedure, the at least one attraction volume defined in part by a point and an axis extending from the point; and while the surgical instrument is in the at least one attraction volume during the THA procedure, provide assistance to movement of the surgical instrument in a direction toward the at least one attraction volume.

Another aspect of the disclosure provides a method of controlling a surgical robot during a total hip arthroplasty (THA) procedure, the method including: detecting a position of the surgical instrument in at least one attraction volume corresponding with a planned trajectory of the surgical instrument for the THA procedure, the at least one attraction volume defined in part by a point and an axis extending from the point; and while the surgical instrument is in the at least one attraction volume during the THA procedure, providing assistance to movement of the surgical instrument in a direction toward the at least one attraction volume.

According to certain embodiments, the at least one attraction volume includes multiple attraction volumes having distinct associated attraction forces.

In some preferred embodiments when the surgical instrument is in at least one attraction volume the controller provides assistance to movement of the surgical instrument in a direction toward a further attraction volume different from the at least one attraction volume.

In some preferred embodiments when the surgical instrument is in at least one attraction volume the controller provides assistance to movement of the surgical instrument through the at least one attraction volume.

In some preferred embodiments when the surgical instrument is in at least one attraction volume the controller provides assistance to movement of the surgical instrument through the at least one attraction volume towards the point in the attraction volume.

According to certain embodiments, a first attraction volume has a lesser attraction force than a second attraction volume, and wherein the second attraction volume is a sub-volume of the first attraction volume.

According to certain embodiments, the point and the axis define a centerline of both the first attraction volume and the second attraction volume.

According to certain embodiments, an operator must exert greater force to remove the surgical instrument from the second attraction volume than the first attraction volume.

According to certain embodiments, the controller is configured to fix the surgical instrument in alignment with the planned trajectory in at least one operating mode.

According to certain embodiments, the surgical instrument includes at least one of a reamer or an impactor.

According to certain embodiments, the controller is further programmed to progressively guide a tip (e.g., distal end) of the surgical instrument to the point and an axis of the surgical instrument to the axis of the attraction volume.

According to certain embodiments, progressively guiding includes providing haptic feedback to guide the tip of the surgical instrument to the point, and subsequently providing haptic feedback to guide the axis of the surgical instrument to the axis of the attraction volume, where the axis of the attraction volume is aligned with the planned trajectory of the surgical instrument for the THA procedure.

According to certain embodiments, the controller is configured to operate in multiple modes during the THA procedure, wherein the controller automatically switches between the operating modes in response to detecting the surgical instrument is aligned with the planned trajectory of the surgical instrument for the THA procedure. In particular embodiments, automatically switching is performed without requiring additional user interaction (e.g., via a command interface). In particular embodiments, operating modes include a free mode, a constrained mode, etc. In particular embodiments, the constrained mode includes a hinge sub-mode, a linear sub-mode, etc.

According to certain embodiments, at least one of the operating modes includes a hinge haptic control mode that limits degrees of freedom of the surgical instrument to permit rotation around the axis thereof.

According to certain embodiments, at least one of the multiple operating modes includes a linear guide haptic control mode that limits degrees of freedom of the surgical instrument to permit translation along the axis.

According to certain embodiments, the at least one attraction volume has an approximately conical shape.

According to certain embodiments, the robot further includes a navigation system for maintaining alignment of the surgical instrument along the planned trajectory for the THA procedure.

According to certain embodiments, the controller is configured to control a reaming portion of the THA procedure by: forcing (e.g., snapping) the surgical instrument into alignment with the planned trajectory for the THA procedure such that a tip of the surgical instrument is coincident with the point, wherein the point defines an initial reaming location; and arresting axial movement of the surgical instrument when the tip reaches a final point that defines a final reaming location, the final reaming location being distal of the initial reaming location. In some cases, after forcing the surgical instrument into alignment with the planned trajectory, the surgical instrument is free to rotate about the axis or to pivot about the point. In further cases, the surgical instrument is free to rotate about the axis or pivot about the point after axial movement has been arrested.

In some examples, the controller arrests movement of the surgical instrument after arriving at the final destination, e.g., no more movement is permitted, even if the user actuates the tool (e.g., reamer spins, but the instrument is fixed so it will not enable further reaming depth). In certain examples, the controller snaps the center point of the volume to the end (e.g., tip) of the tool (e.g., reamer), defining an initial reaming location. At the initial reaming location, the controller enables the user to pivot/rotate the instrument around the point, and allows for reaming, which removes tissue linearly and after reaming, arrives at a second center point (final center point), where the controller arrests axial movement of the surgical instrument (e.g., still enabling rotation/pivoting about point).

According to certain embodiments, the surgical robot further includes: an end effector for holding the surgical instrument, wherein the end effector enables coupling of distinct surgical instruments for the THA procedure.

According to certain embodiments, a system includes the surgical robot, the system further including: a reference element on the surgical instrument; and a user interface enabling a user to monitor the position of the surgical instrument, as indicated in part by the reference element, during the THA procedure.

These and other aspects, advantages and salient features of the invention will become apparent from the following detailed description, which, when taken in conjunction with the annexed drawings, where like parts are designated by like reference characters throughout the drawings, disclose embodiments of the invention.

### BRIEF DESCRIPTION OF THE DRAWINGS

Aspects of the present disclosure are illustrated by way of example and are not limited by the accompanying drawings. In the drawings:
**FIG. 1** is an overhead view of a surgical system arranged during a surgical procedure in a surgical room which includes a camera tracking system for computer assisted navigation during surgery, and a surgical robot for robotic assistance according to some embodiments of the present disclosure.
**FIG. 2** illustrates the camera tracking system and the surgical robot of **FIG. 1****,** positioned relative to a patient according to some embodiments of the present disclosure.
**FIG. 3** further illustrates the camera tracking system and the surgical robot of **FIGS. 1-2****,** configured according to some embodiments of the present disclosure.
**FIG. 4** illustrates a block diagram of a surgical system that includes an extended reality headset, a computer platform, imaging devices, and a surgical robot, which are configured to operate according to some embodiments of the present disclosure.
**FIG. 5** illustrates a flowchart of a workflow during an intra-operative portion of a total hip arthroplasty (THA) surgery, in accordance with some embodiments of the present disclosure.
**FIG. 6** illustrates a flowchart of a patient preparation process before registration, in accordance with some embodiments of the present disclosure.
**FIG. 7** illustrates a radiographic inclination angle measured in the coronal plane of the patient, in accordance with some embodiments of the present disclosure.
**FIG. 8** illustrates a radiographic version angle measured relative to the coronal plane of the patient, in accordance with some embodiments of the present disclosure.
**FIG. 9** illustrates different views of landmarks and axes for registration of a functional pelvic plane (FPP) and an anterior pelvic plane (APP) of a patient, in accordance with some embodiments of the present disclosure.
**FIG. 10** provides a flow chart illustrating a workflow for robot-assisted preparations of one or more bones such as, e.g., the acetabulum, by reaming prior to implant placement during THA surgery.
**FIG. 11** provides a perspective view of a portion of a surgical robot, arm, end effector, and reamer, and certain movements thereof that are permitted in a force control mode of operation, in accordance with some embodiments of the present disclosure.
**FIG. 12** provides a perspective view of a portion of a surgical robot, arm, end effector, and reamer, and certain movements thereof that are permitted in a point rotate control mode of operation, in accordance with some embodiments of the present disclosure.
**FIG. 13** provides a perspective view of a portion of a surgical robot, arm, end effector, and reamer, and certain movements thereof that are permitted in axis rotate control mode of operation, in accordance with some embodiments of the present disclosure.
**FIG. 14** provides a perspective view of a portion of a surgical robot, arm, end effector, and reamer, and certain movements thereof that are permitted in a translate control mode of operation, in accordance with some embodiments of the present disclosure.

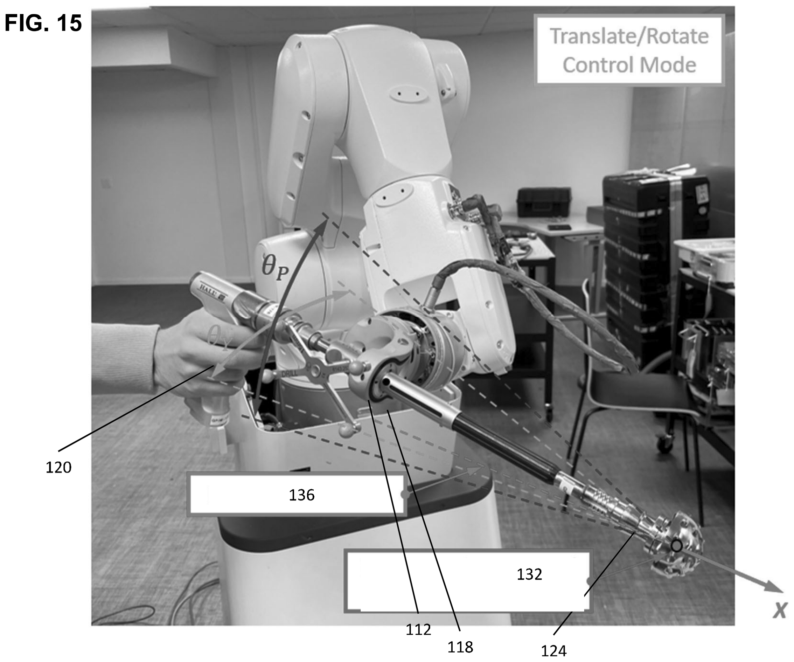

**FIG. 15** provides a perspective view of a portion of a surgical robot, arm, end effector, and reamer, and certain movements thereof that are permitted in a translate/rotate control mode of operation, in accordance with some embodiments of the present disclosure.
**FIG. 16** provides a perspective view of a portion of a reamer, and certain movements thereof that are permitted in a translate/rotate control mode, in accordance with some embodiments of the present disclosure.
**FIG. 17** provides a flow chart illustrating a workflow for robotic trialing of the acetabulum shell during THA surgery, in accordance with various implementations.
**FIG. 18** provides a flow chart illustrating workflow for robotic placement of an acetabular shell implant during THA surgery, in accordance with various implementations.
**FIG. 19** provides a perspective view of a surgical instrument and attraction volume(s) to aid in performing a THA surgery according to various implementations.
**FIG. 20** is a close-up perspective view of a portion of a surgical instrument interacting with attraction volumes according to various implementations.
**FIG. 21** is another close-up perspective view of a portion of a surgical instrument interacting with attraction volumes according to various implementations.
**FIG. 22** is perspective view of a robot arm holding a surgical instrument in a first operating mode according to various implementations.
**FIG. 23** is perspective view of a robot arm holding a surgical instrument in a second operating mode according to various implementations.
**FIG. 24** is a flow diagram illustrating processes in a method according to various implementations.
**FIG. 25** is a flow diagram illustrating processes in a method according to various additional implementations.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

### DETAILED DESCRIPTION OF THE INVENTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "attached," "supported," and "coupled" and variations thereof are used broadly and encompass both direct and indirect mountings, connections, attachments, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

The present application is related to (1) Patent App. No. 15/180,126, filed June 13, 2016 (U.S. 10,842,453), (2) Patent App. No. 15/157,444, filed May 18, 2016 (U.S. Pub. No. 2016/0256225), (3) Patent Application No. 18/743,685 (Docket ROBOT.143.0005/IDR-24-008), (4) Patent Application No. 18/743,388 (Docket ROBOT.143.0002/ IDR-23-131), (5) Patent Application No. 18/743,647 (Docket ROBOT.143.0004/ IDR-23-149), (6) Patent Application No. 18/743,615 (Docket ROBOT.143.0003/ IDR-23-151), and (7) US Patent No. 10,058,394 (App. No. 14/815,198), each of which is incorporated herein by reference.

Robotic surgery systems and workflows associated therewith may provide improved outcomes in surgeries such as, e.g., THA surgeries compared to more traditional approaches. For example, robotic surgery systems may provide additional accuracy and force assistance when preparing the acetabulum, and additional accuracy and alignment when trialing and placing implants. In certain embodiments, e.g., including implant location preparation, implant trialing, and/or implant placement, a surgical robot can be configured to provide attraction force-based (or haptic-based) feedback to guide the user through the THA procedure. In particular implementations, a robot controller coupled with a robot arm is programmed to detect a position of the surgical instrument in at least one attraction volume corresponding with a planned trajectory of the surgical instrument for the THA procedure. The at least one attraction volume can be defined in part by a point and an axis extending from the point. The robot controller is also programmed, while the surgical instrument is in the at least one attraction volume during the THA procedure, to provide an attraction force to move the surgical instrument in a direction toward the at least one attraction volume. As described in US 10,058,394 ("Robot Arm and Methods of Use," issued August 28, 2018 and incorporated by reference in its entirety), attractive force-based movement of the surgical instrument can be controlled via a robot arm, e.g., at the end effector. More particularly, a controller can control one or more motors operable to move the robot arm and/or end effector to provide guidance and/or resistance to the user of the surgical instrument during the THA procedure. Aspects of the disclosed embodiments are discussed below.

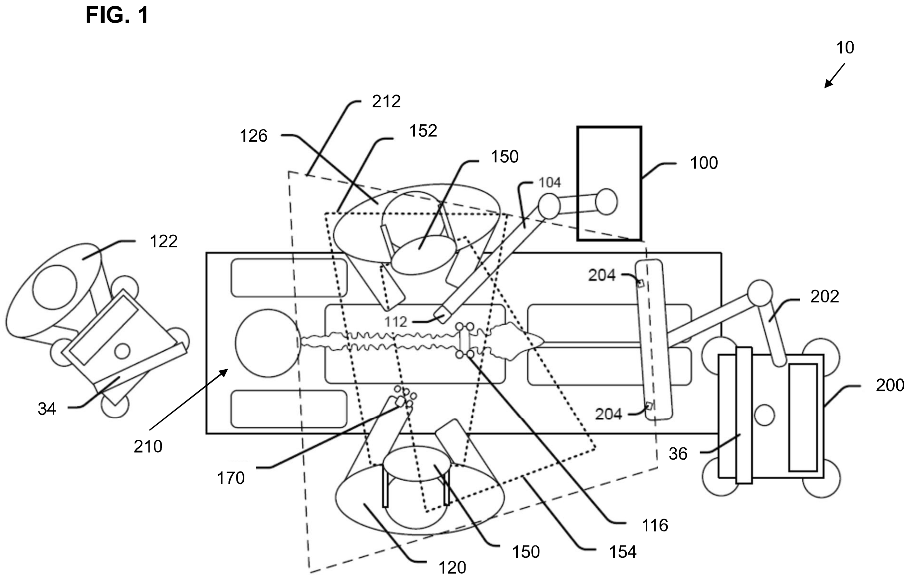

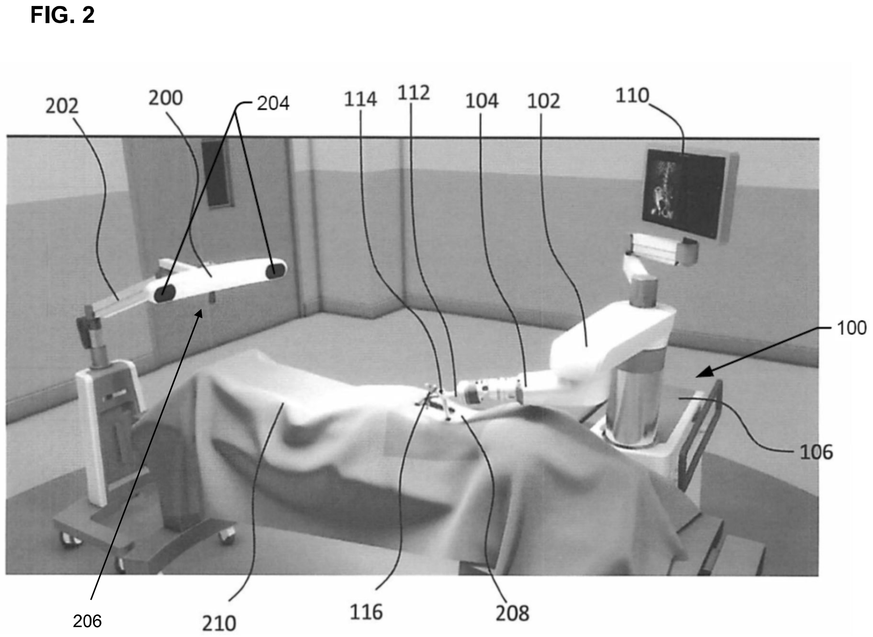

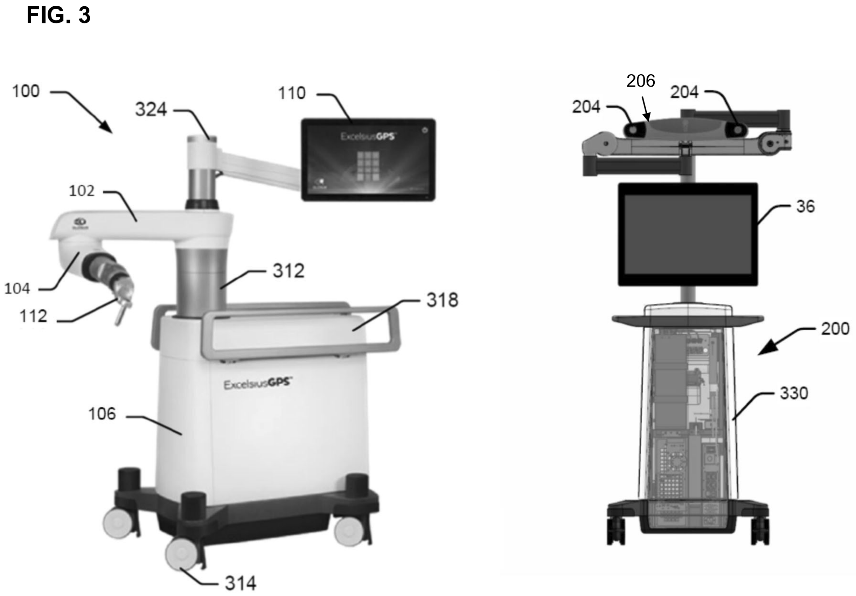

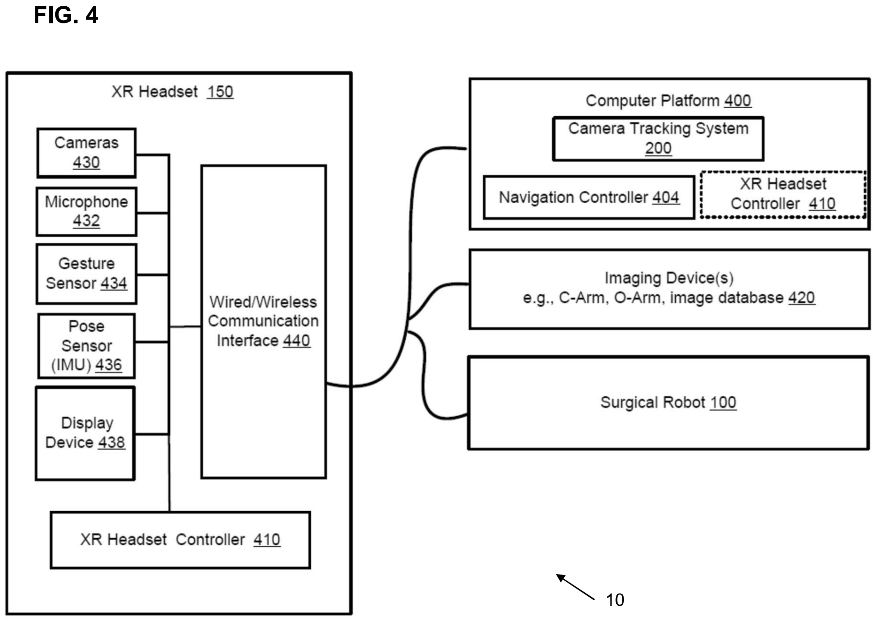

**FIG. 1** is an overhead view of a surgical system 10 arranged during a surgical procedure in a surgical or operating room. The system 10 includes a camera tracking system 200 for computer assisted navigation during surgery and may further include a surgical robot 100 for robotic assistance according to some embodiments. **FIG. 2** illustrates the camera tracking system 200 and the surgical robot 100 positioned relative to a patient according to some embodiments. **FIG. 3** further illustrates the camera tracking system 200 and the surgical robot 100 configured according to some embodiments. **FIG. 4** illustrates a block diagram of a surgical system 10 that includes an extended reality (XR) headset 150, a computer platform 400, imaging devices 420, and the surgical robot 100 which are configured to operate according to some embodiments.

The camera tracking system 200 (**FIGS. 1-4**) in some cases includes an intraoperative imaging system, that can include distinct imaging modalities. These imaging modalities may include one or more of fluoroscopy, 2D Radiography, and Cone-beam computed tomography (CBCT). Fluoroscopy is a medical imaging technique that shows a continuous X-ray image on a monitor, much like an X-ray movie. 2Dclaims Radiography is an imaging technique that uses X-rays to view the internal structure of a non-uniformly composed and opaque object such as the human body. CBCT (or, cone beam 3D imaging or C-arm CT), is a medical imaging technique consisting of X-ray computed tomography where the X-rays are divergent, forming a cone. The camera tracking system 200 is capable of: (1) capturing three-dimensional (3D) images (e.g., CT, CBCT, MCT, PET, Angiogram, MRI, ultrasound, etc.), (2) capturing two-dimensional (2D) images (e.g., fluoroscopy, digital radiography, ultrasound, etc.), and (3) containing an integrated or detachable navigation array having tracking markers (e.g., NIR retroreflective, NIR LED, visible, etc.), which is calibrated to the image space of the 2D and 3D images.

The system 10, including surgical robot 100, is capable of: (1) using registered 2D and/or 3D images for surgical planning, navigation, and guidance in a variety of workflows (e.g., intraoperative 3D, intraoperative 2D, preoperative 3D to 2D, and intraoperative 3D to 2D, etc.); and (2) containing a camera tracking system 200 capable of tracking markers (e.g., NIR retroreflective, NIR LED, visible, etc.). In some cases, as noted herein, a dynamic reference base (DRB) (or patient reference array) 116 is (1) capable of rigidly attaching to the patient anatomy, and (2) contains an array of tracking markers (e.g., NIR retroreflective, NIR LED, visible, etc.).

The XR headsets 150 may be configured to augment a real-world scene with computer generated XR images while worn by personnel in the operating room. The XR headsets 150 may be configured to provide an augmented reality (AR) viewing environment by displaying the computer generated XR images on a see-through display screen that allows light from the real-world scene to pass therethrough for combined viewing by the user. Alternatively, the XR headsets 150 may be configured to provide a virtual reality (VR) viewing environment by preventing or substantially preventing light from the real-world scene from being directly viewed by the user while the user is viewing the computer-generated AR images on a display screen. The XR headsets 150 can be configured to provide both AR and VR viewing environments. Thus, the term XR headset encompasses both or either of an AR headset or a VR headset.

With continuing reference to **FIGS. 1-4****,** the surgical robot 100 may include, for example, one or more robot arms 102, 104, a display 110, an end effector 112, for example, including a guide tube 118, and an end effector reference element 114 which can include one or more tracking fiducials. A patient reference element (or DRB) 116 (shown in **FIG. 1**) has a plurality of tracking fiducials and is secured directly to the patient 210. For example, a navigated pelvis DRB marker array may be placed intra-incision or extra-incision with the help of cortical pins drilled into the pelvic bone. In some embodiments, the DRB is oriented to be visible by the tracking camera(s) 204 (e.g., a stereoscopic tracking camera) installed on the camera tracking system 200 and/or the XR headset 150. A reference element 170 is attached to or formed on an instrument, surgical tool, surgical implant device, etc.

The camera tracking system 200 includes tracking cameras 204 which may be spaced apart to provide stereo cameras configured with partially overlapping fields-of-view. The camera tracking system 200 can have any suitable configuration of arm(s) 202 to move, orient, and support the tracking cameras 204 in a desired location, and may contain at least one processor operable to track the location of an individual fiducial and pose of an array of fiducials of a reference element.

As used herein, the term "pose" refers to the location (e.g., along three orthogonal axes, e.g., the x-, y-, and z-axes) and/or the rotation angle (e.g., about the three orthogonal axes) of fiducials (e.g., DRB) relative to another fiducial (e.g., surveillance fiducial) and/or to a defined coordinate system (e.g., camera coordinate system, navigation coordinate system, etc.). A pose may therefore be defined based on only the multidimensional location of the fiducials relative to another fiducial and/or relative to the defined coordinate system, based on only the multidimensional rotational angles of the fiducials relative to the other fiducial and/or to the defined coordinate system, or based on a combination of the multidimensional location and the multidimensional rotational angles. The term "pose" therefore is used to refer to location, rotational angle, or combination thereof of, e.g., an instrument reference element 170, a patient reference element 116, or the like.

The tracking cameras 204 may include, e.g., infrared cameras (e.g., bifocal or stereophotogrammetric cameras) operable to identify, for example, active and passive tracking fiducials for single fiducials (e.g., a surveillance fiducial) and reference elements which can be formed on or attached to the patient 210 (e.g., patient reference element or DRB 116), end effector 112 (e.g., end effector reference element 114), XR headset(s) 150 worn by a surgeon 120 and/or a surgical assistant 126, etc. in a given measurement volume of a camera coordinate system while viewable from the perspective of the tracking cameras 204. The tracking cameras 204 may scan the given measurement volume and detect light that is emitted or reflected from the fiducials in order to identify and determine locations of individual fiducials and poses of the reference elements in three-dimensions. For example, active reference elements may include infrared-emitting fiducials that are activated by an electrical signal (e.g., infrared light emitting diodes (LEDs)), and passive reference elements may include retroreflective fiducials that reflect infrared light (e.g., they reflect incoming IR radiation into the direction of the incoming light), for example, emitted by illuminators on the tracking cameras 204 or other suitable device.

The XR headsets 150 may each include tracking cameras (e.g., spaced apart stereo cameras) that can track the location of a surveillance fiducial and poses of reference elements within the XR camera headset fields of view (FOVs) 152 and 154, respectively. Accordingly, as illustrated in **FIG. 1****,** the location of the surveillance fiducial and the poses of reference elements on various objects such as, e.g., instrument reference element 170 and patient reference element 116, can be tracked while in the FOVs 152 and 154 of the XR headsets 150 and/or a FOV 212 of the tracking cameras 204.

**FIGS. 1** **and** **2** illustrate a potential configuration for the placement of the camera tracking system 200 and the surgical robot 100 in an operating room environment. Computer assisted navigated robotic surgery can be provided by the surgical robot 100, the camera tracking system 200 controlling the XR headsets 150 and/or other displays 34, 36, and 110 to display surgical procedure navigation information.

The camera tracking system 200 may operate using tracking information and other information provided by multiple XR headsets 150 such as inertial tracking information and optical tracking information (frames of tracking data). The XR headsets 150 operate to display visual information and may play-out audio information to the wearer. This information can be from local sources (e.g., the surgical robot 100), imaging devices 420 **(****FIG. 4**), remote sources (e.g., patient medical image database), and/or other electronic equipment. The camera tracking system 200 may track fiducials in 6 degrees-of-freedom (6 DOF) relative to three axes of a 3D coordinate system and rotational angles about each axis. The XR headsets 150 may also operate to track hand poses and gestures to enable gesture-based interactions with "virtual" buttons and interfaces displayed through the XR headsets 150, and can also interpret hand or finger pointing or gesturing as various defined commands. Additionally, the XR headsets 150 may have a 1-10x magnification digital color camera sensor called a digital loupe. In some embodiments, one or more of the XR headsets 150 are minimalistic XR headsets that display local or remote information but include fewer sensors and are therefore more lightweight.

An "outside-in" machine vision navigation bar 206 supports the tracking cameras 204 and may include a color camera. The machine vision navigation bar generally has a more stable view of the environment because it does not move as often or as quickly as the XR headsets 150 while positioned on wearers' heads. The patient reference element (or, DRB) 116 is generally rigidly attached to the patient 210 with stable pitch and roll relative to gravity. This local rigid patient reference 116 can serve as a common reference for reference frames relative to other tracked elements, such as a reference element 114 on the end effector 112, instrument reference element 170, and reference elements on the XR headsets 150.

In some embodiments, at the end of the end effector 112, instruments are connected to perform operations such as resection, reaming, and implant placement.

The surgical robot 100 may be positioned near or next to patient 210 as shown in **FIGS. 1-2****.** The robot 100 can be positioned at any suitable location near the patient 210 depending on the area of the patient 210 undergoing the surgical procedure. The camera tracking system 200 may be separate from the robot system 100 and positioned at the foot of patient 210. This location allows the tracking camera 200 to have a direct visual line of sight to the surgical area 208, e.g., the hip area **(****FIG. 2**). In the configuration shown in **FIG. 1****,** the surgeon 120 may be positioned across from the robot 100, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. An anesthesiologist 122, nurse, or scrub tech can operate equipment which may be connected to display information from the camera tracking system 200 on a display 34 **(****FIG. 1**).

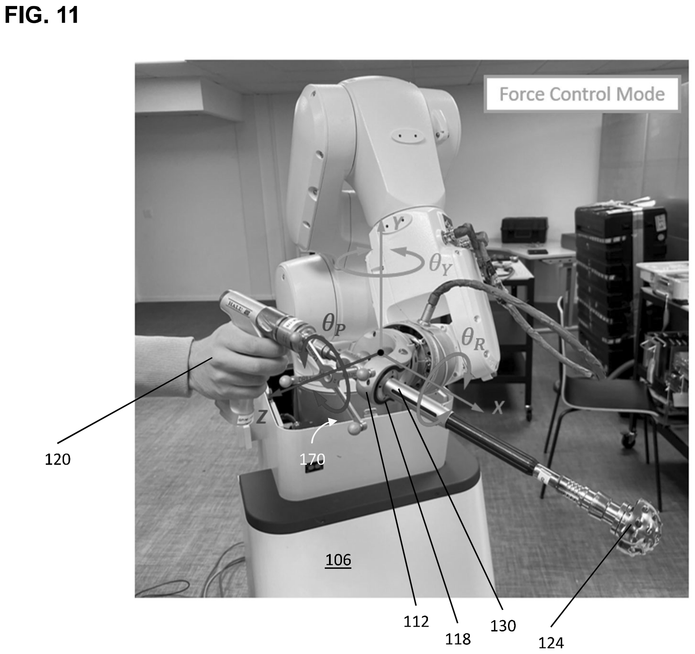

With respect to the other components of the robot 100, the display 110 can be attached to the surgical robot 100 or in a remote location. The end-effector 112 may be coupled to the robot arm 104 and be controlled by at least one motor. An upper arm 102 may further couple the arm 104 to the column 312 of the robot 100. In some embodiments, end effector 112 includes a guide tube 118 (e.g., **FIG. 11**), which is configured to receive and orient a surgical instrument, tool, or implant used to perform a surgical procedure on the patient 210. For example, the end effector 112 is adapted to receive (e.g. through a guide tube 118) a surgical instrument or a portion thereof, to removably couple to the instrument, and to manipulate the instrument such as by translating and rotating the instrument. In some other embodiments, the end-effector 112 includes a passive structure guiding a saw blade (e.g., sagittal saw) along a defined cutting plane.

As used herein, the term "end effector" is used interchangeably with the terms "end effectuator" and "effectuator element." The term "instrument" is used in a non-limiting manner and can be used interchangeably with "tool" and "implant" to generally refer to any type of device that can be used during a surgical procedure in accordance with embodiments disclosed herein. The more general term, device, can also refer to structure of the end effector, etc. Example instruments, tools, and implants include, without limitation, reamer constructs, drills, screwdrivers, saws, dilators, retractors, probes, implant inserters, and implant devices such as shells and trial shells, screws, spacers, interbody fusion devices, plates, rods, etc. Although generally shown with a guide tube 118, it will be appreciated that the end-effector 112 may be replaced with any suitable instrumentation for use in surgery. In some embodiments, end-effector 112 can comprise any known structure for effecting the movement of the surgical instrument in a desired manner.

The surgical robot 100 is operable to control the translation and orientation of the end-effector 112. The robot 100 may move the end-effector 112 under computer control along x-, y-, and z-axes, for example. The end-effector 112 can be configured for selective rotation about one or more of the x-, y-, and z-axes, and a Z Frame axis, such that one or more of the Euler Angles (e.g., roll, pitch, and/or yaw) associated with the end effector 112 can be selectively computer controlled. In some embodiments, selective control of the translation and orientation of end effector 112 and associated surgical instrument can permit performance of medical procedures with significantly improved accuracy compared to conventional robots that utilize, for example, a 6 DOF robot arm comprising only rotational axes. For example, the surgical robot 100 may be used to operate on patient 210, and robot arm 104 can be positioned above the body of patient 210, with end-effector 112 selectively angled relative to the z-axis toward the body of patient 210.

In some example embodiments, the XR headset(s) 150 can be controlled to dynamically display an updated graphical indication of the pose of the surgical instrument so that the user, e.g., surgeon 120, can be aware of the pose of the surgical instrument at all times during the procedure.

In some further embodiments, surgical robot 100 can be operable to correct the path of a surgical instrument guided by the robot arm 104 if the surgical instrument strays from the selected, preplanned, or defined trajectory. The surgical robot 100 can be operable to permit stoppage, modification, and/or manual control of the movement of end effector 112 and/or the surgical instrument. Thus, in use, a surgeon 120 or other user can use the surgical robot 100 as part of computer assisted navigated surgery, and has the option to stop, modify, or manually control the autonomous or semi-autonomous movement of the end-effector 112 and/or the surgical instrument.

Fiducials or reference elements can be formed on or connected to robot arms 102 and/or 104, the end effector 112 (e.g., end effector element 114 in **FIG. 2**), and/or a surgical instrument (e.g., instrument element 170) to enable tracking of poses in a defined coordinate system, e.g., such as in six degrees of freedom (DOF) along three orthogonal axes and rotation about the axes. The reference elements 114, 116, 170 enable each of the marked objects (e.g., the end-effector 112, the patient 210, and the surgical instruments, respectively) to be tracked by the tracking camera 200, and the tracked poses can be used to provide navigated guidance during a surgical procedure and/or to control movement of the surgical robot 100 for guiding the end effector 112 and/or an instrument manipulated by the end effector 112. The instrument manipulated by the end effector 112 may include, e.g., a reamer 124 or an inserter adapted to insert an implant.

Referring to **FIG. 3** the surgical robot 100 may include a display 110, upper arm 102, lower arm 104, end effector 112, vertical column 312, casters 314, a table 318, and ring 324 which uses lights to indicate statuses and other information. Cabinet 106 may house electrical components of surgical robot 100 including, but not limited to, a battery, a power distribution module, a platform interface board module, and a computer. The camera tracking system 200 may include a display 36, tracking cameras 204, arm(s) 202 **(****FIG. 1**), a computer housed in cabinet 330, and other components.

In computer assisted navigated surgeries, perpendicular 2D scan slices, such as axial, sagittal, and/or coronal views of patient anatomical structure are displayed to enable user visualization of the patient's anatomy alongside the relative poses of surgical instruments. An XR headset or other display can be controlled to display one or more 2D scan slices of patient anatomy along with a 3D graphical model of anatomy. The 3D graphical model may be generated from a 3D scan of the patient, e.g., by a CT scan device, and/or may be generated based on a baseline model of anatomy which isn't necessarily formed from a scan of the patient.

### Example Surgical System

**FIG. 4** illustrates a block diagram of a surgical system 10 that includes a surgical robot 100, a computer platform 400 including, *inter alia,* the camera tracking system 200, imaging device(s) 420, and XR headset(s) 150 which are configured to operate as described herein, according to some embodiments.

The imaging device(s) 420 may include a C-arm imaging device, an O-arm imaging device, other imaging device, and/or a patient image database of 2D and/or 3D images. The XR headset 150 provides a human interface for performing navigated surgical procedures. The XR headset 150 can be configured to provide functionalities, e.g., via the computer platform 400, that include without limitation any one or more of: identification of hand gesture-based commands, and display of XR graphical objects on a display device 438 of the XR headset 150 and/or another display device. The display device 438 may include a video projector, flat panel display, etc. The user may view the XR graphical objects as an overlay anchored to particular real-world objects viewed through a see-through display screen. The XR headset 150 may additionally or alternatively be configured to display on the display device 438 video streams from cameras mounted to one or more XR headsets 150 and other cameras.

Electrical components of the XR headset 150 can include a plurality of cameras 430, a microphone 432, a gesture sensor 434, a pose sensor (e.g., inertial measurement unit (IMU)) 436, the display device 438, and a wireless/wired communication interface 440. The cameras 430 of the XR headset 150 may be visible light capturing cameras, near infrared capturing cameras, or a combination of both.

The cameras 430 may be configured to operate as the gesture sensor 434 by tracking for identification user hand gestures performed within the field-of-view of the camera(s) 430. Alternatively, the gesture sensor 434 may be a proximity sensor and/or a touch sensor that senses hand gestures performed proximately to the gesture sensor 434 and/or senses physical contact, e.g., tapping on the sensor 434 or its enclosure. The pose sensor 436, e.g., IMU, may include a multi-axis accelerometer, a tilt sensor, and/or another sensor that can sense rotation and/or acceleration of the XR headset 150 along one or more defined coordinate axes. Some or all of these electrical components may be contained in a head-worn component enclosure or may be contained in another enclosure configured to be worn elsewhere, such as on the hip or shoulder.

As explained above, the surgical system 10 includes the camera tracking system 200 which may be connected to a computer platform 400 for operational processing and which may provide other operational functionality including a navigation controller 404 and/or an XR headset controller 410. The surgical system 10 may further include the surgical robot 100. The navigation controller 404 can be configured to provide visual navigation guidance to an operator for moving and positioning a surgical tool relative to patient anatomical structure based on a surgical plan, e.g., from a surgical planning function, defining where a surgical procedure is to be performed using the surgical tool on the anatomical structure and based on a pose of the anatomical structure determined by the camera tracking system 200. The navigation controller 404 may be further configured to generate navigation information based on a target pose for a surgical tool, a pose of the anatomical structure, and a pose of the surgical tool and/or an end effector 112 of the surgical robot 100. The navigation information may be displayed through the display device 438 of the XR headset 150 and/or another display device to indicate where the surgical tool and/or the end effector 112 of the surgical robot 100 should be moved to perform a surgical procedure according to a defined surgical plan.

The electrical components of the XR headset 150 can be operatively connected to the electrical components of the computer platform 400 through the wired/wireless interface 440. The electrical components of the XR headset 150 may be operatively connected, e.g., through the computer platform 400 or directly connected, to various imaging devices 420, e.g., the C-arm imaging device, the O-arm imaging device, other imaging device(s), the patient image database, and/or to other medical equipment through the wired/wireless interface 440.

The surgical system 10 may include a XR headset controller 410 that at least partially resides in the XR headset 150, the computer platform 400, and/or another system component connected via wired cables and/or wireless communication links. Various functionality may be provided by software executed by the XR headset controller 410. The XR headset controller 410 is configured to receive information from the camera tracking system 200 and the navigation controller 404, and to generate an XR image based on the information for display on the display device 438.

The XR headset controller 410 can be configured to operationally process frames of tracking data from the cameras 430 (tracking cameras), signals from the microphone 432, and/or information from the pose sensor 436 and the gesture sensor 434, to generate information for display as XR images on the display device 438 and/or for display on other display devices for user viewing. Thus, the XR headset controller 410 as illustrated as a circuit block within the XR headset 150 is to be understood as being operationally connected to other illustrated components of the XR headset 150 but not necessarily residing within a common housing or being otherwise transportable by the user. For example, the XR headset controller 410 may additionally or alternatively reside within the computer platform 400 which, in turn, may reside within the cabinet 330 of the camera tracking system 200, the cabinet 106 of the surgical robot 100, etc.

### Exemplary Patient Registration Workflows

In some embodiments of the present disclosure, the system 10, e.g., computer platform 400, may perform one of a number of available workflows to register a patient to the surgical system 10 prior to surgery. The workflows may further include isolating a target area for the surgical procedure from non-target surgical areas. In one example, the target surgical area may include the acetabulum, and the non-target surgical area may include the femur.

In one embodiment, the workflow may be an imageless workflow in which no pre-operative images are used. Instead, information about the patient anatomy in the operating room (OR) can be obtained by the surgeon measuring key parameters of the patient's bone using the system as described herein. For example, the computer platform 400 of the system 10 operates to identify the locations of landmarks (e.g., points, axes, and/or surfaces) on the bone and register the locations either concurrently with the identification or thereafter. The locations can be used to define reference plane(s) (e.g., anterior pelvic plane (APP) and/or functional pelvic plane (FPP)) which, in turn, are used to plan implants and navigate the robot and surgical instruments for THA surgical procedures.

In some embodiments, the only pre-operative use case associated with the imageless workflow may be the initial patient assessment. The surgeon may assess the patient's mobility and health status with assistance from sensors (e.g., sensors made by Globus Medical which are attached to the leg), physical exercises, and/or clinical surveys to determine if THA is recommended. Gathered data may then be stored and processed by the system before being analyzed by the surgeon to facilitate a final decision. Subsequently, the data may be reused by an application (e.g., surgery planning application by Globus Medical) to establish the most appropriate implant surgical plan.

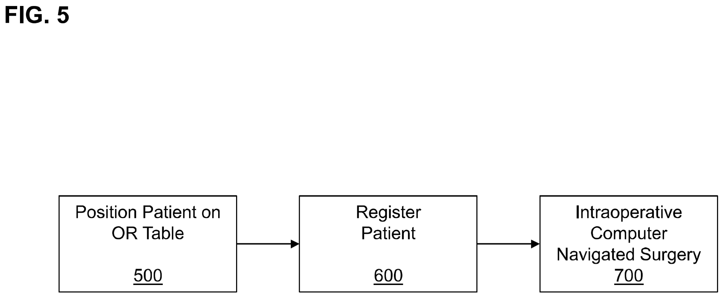

**FIG. 5** illustrates a flowchart for an imageless workflow during an intra-operative portion of a THA surgery, in accordance with some embodiments of the present disclosure. In some embodiments, after positioning the patient on the operating room table (process 500), some of the operations discussed above and below may be performed during process 600 to register a patient and before another process 700 for intraoperative computer navigated surgery. In the case of a hip, a pelvis or acetabulum of the patient is registered in the tracking coordinate system of the camera tracking system 200. As shown in **FIG. 1****,** the pelvis or acetabulum is registered in the optical coordinate system. In one embodiment, the registration is done in an imageless modality without the use of any medical images such as X-rays or CT images from an imaging device. As noted herein, in other embodiments, registration is performed using one or more pre-operative X-ray images and/or CT images.

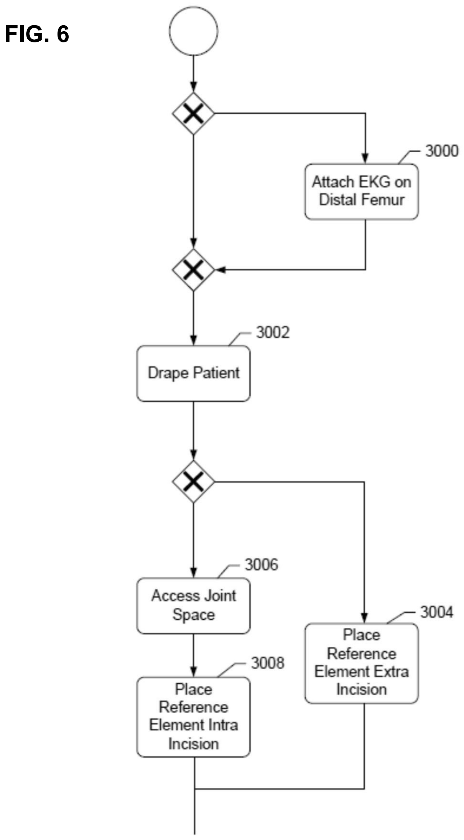

**FIG. 6** illustrates a flowchart of a patient preparation process before registration, in accordance with some embodiments of the present disclosure.

The patient preparation process may begin with a patient being positioned in a lateral or supine position on the OR table. The patient's body is prepared for registration. Optionally, in process 3000, an EKG/ECG patch electrode is attached on or adjacent a distal end of the patient's femur. The EKG/ECG patch electrode may be placed on the center of the patella or slightly inferior to the center. In some embodiments, the patch location is in line with the anatomic axis of the femur. This patch may be used to acquire the most distal point of the femur under the drape at a later stage. This patch may also be used to track the femur in space (e.g., when the patient's leg is moved during surgery) and may also be used to assist in measuring the patient's leg length. However, in some embodiments, this operation (process 3000) is skipped.

In some embodiments, the EKG/ECG patch electrode includes an adhesive patch that is removably attachable to the patient. In some embodiments, the patch may be black or dark to be more visible to the tracking camera. In other embodiments, the patch and patch electrodes are not visible by the tracking camera as they are under a drape. The patch geometry (like a nipple) will help the surgeon to always touch a single point on or adjacent the distal part of the femur (anterior patella region) with a navigated stylus/instrument which is trackable by the tracking camera. This ensures that the surgeon always collects the same point to measure the leg length or medio-lateral offset.

In process 3002, the patient body is draped. Then, depending on the surgeon's technique, the navigated pelvis DRB is placed intra-incision (processes 3006-3008) or extra-incision (process 3004) with the help of cortical pins drilled into the pelvic bone. In some embodiments, the DRB is oriented to be visible to the tracking camera(s), e.g., a stereoscopic tracking camera installed on the camera tracking system 200 **(****FIG. 1**) or the XR headset 150 **(****FIG. 1**). In one embodiment, the operation to place the DRB intra-incision, includes using the system to track and navigate access to the joint space (process 3006) and placing the reference element intra-incision. In an alternative embodiment, the reference element is placed extra-incision (process 3004) and the system does not necessarily need to be used to track and navigate access to the joint space.

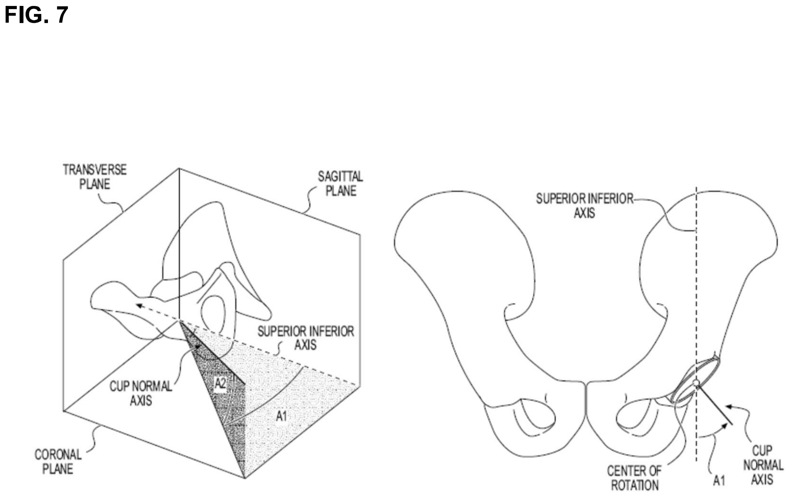

After the reference element or DRB has been placed intra-incision or extra-incision, data points and axes can be collected on the patient anatomy with the assistance of navigated instruments and using the pelvis DRB coordinate system as a spatial reference. In addition to this, two pelvic reference planes can be established to plan placement of implants by measuring angular deviations such as inclination and version of the acetabular cup implant as shown in **FIGS. 7-8****.**

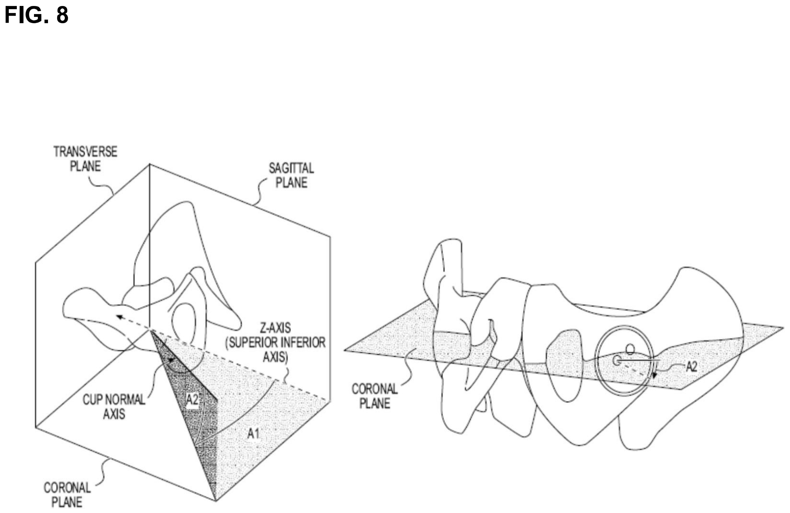

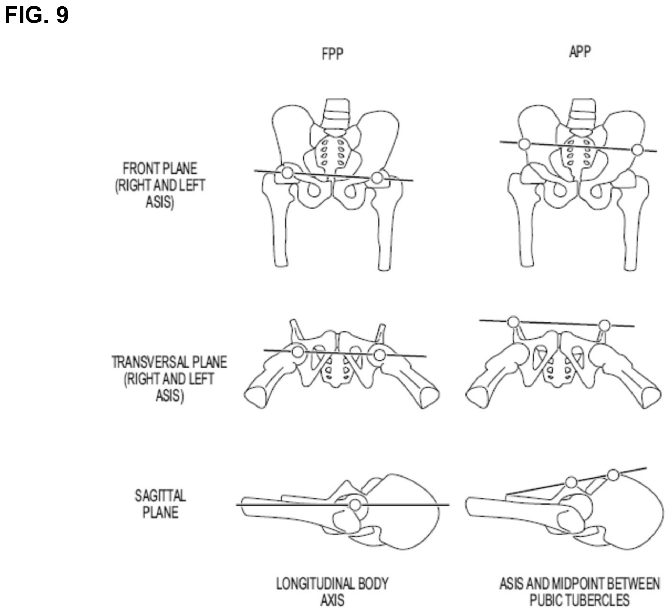

**FIG. 7** illustrates a radiographic inclination angle measured in the coronal plane of the patient, in accordance with some embodiments of the present disclosure. In some embodiments, the surgeon may use a navigated instrument to palpate or paint the surface of the acetabular cavity of the patient to determine a center of rotation of the acetabulum. **FIG. 8** illustrates a radiographic version angle measured relative to the coronal plane of the patient, in accordance with some embodiments of the present disclosure. The two pelvic reference planes (or coronal or frontal planes), the anterior pelvic plane (APP) and functional pelvic plane (FPP), are determined or defined using different landmarks and axes as shown on **FIG. 9** and described in further detail below.

It is to be understood herein that although the user interfaces and associated operations are described as being performed in a certain sequence, they may be performed in other sequences while still being within disclosed embodiments. Moreover, it is not necessary that all of the user interfaces and/or described operations be performed. Instead, fewer operations may be performed while still being within disclosed embodiments. Further, additional registration approaches can include image-based and imageless workflows. Combinations of these registration approaches are also possible in keeping with the various disclosed embodiments.

During a patient registration procedure, landmarks used to register patient anatomy can be extracted using either single point palpation collection or surface painting (resulting in a point cloud of locations). **FIG. 9** illustrates different views of landmarks and axes for registration of the FPP and APP of a patient, in accordance with some embodiments of the present disclosure. The landmarks and axes used to register the APP and FPP planes are described in more detail in US Patent Application No. 18/430,077 (Docket No. ROBOT.134.0002), previously incorporated by reference herein.

Further, US Patent Application No. 18/430,077 (Docket No. ROBOT.134.0002) discloses processes for registration of a pelvic acetabulum of a patient (including painting the acetabular cavity), in accordance with various embodiments of the present disclosure. For example, to define the APP and FPP origins, the pelvic acetabular center of rotation can be determined after removing the femoral head of the patient from the acetabular cavity. The acetabular cavity may be made accessible by cutting the femoral neck and by removing the femoral head from the acetabular cavity. In some embodiments, a cork screw instrument may be used to remove the femoral head from the acetabular cavity.

The surface of the acetabular cavity can then be painted using the navigated instrument (e.g., a stylus). For example, the surgeon may use the navigated instrument (e.g., stylus) to palpate the surface of the acetabular cavity, as the tracking camera measures the position of a ball on the end of the stylus in a continuous way. This process provides a cloud of points for the measured positions (locations). At the same time, the tracking camera may also monitor and track the pose of the patient DRB 116 attached to the pelvis such that the pose of the stylus can be tracked relative to the pose of the patient DRB. Alternatively, the surgeon may subsequently measure a predefined number or percentage of points by palpating them one-by-one. Based on these points and the tracking data of the stylus and patient DRB 116, the center of rotation of the pelvic acetabular cavity is determined. Additionally, based on these points, the surface of the acetabular cavity may be registered in the system and/or a 3D model may be generated or modified based on these points. Next, the acetabular cavity shape can be recreated (e.g., in a 3D model) by the system based on the measured cloud of points and using other algorithms, e.g., for outlier removals and surface fitting.

While certain imageless approaches are described herein and in US Patent Application No. 18/430,077 (Docket No. ROBOT.134.0002), previously incorporated by reference herein, other example methods of performing imageless and image-based registration of the pelvis to the tracking coordinate system of the tracking system (e.g. optical coordinate system). These methods may also be used to, e.g., determine a native center of rotation of the acetabulum, derive or define an FPP, and derive or define an APP. Registration may allow a navigation system or robotic system to track any navigated instrument or end effector 112 or any tool attached to the end effector 112 relative to the pelvis as tracked by a patient dynamic reference base 116 attached to the pelvis. Various registration methods described herein can be combined in keeping with various disclosed embodiments.

For example, in one imageless method, an APP is derived by either touching various known points (e.g., left and right anterior superior iliac spine (ASIS) and pubic symphysis) with a navigated instrument, or by a physician lining up a plane or axis defined by the navigated instrument along or parallel to the APP. With the center of rotation and APP determined, the system (either a navigation system or a combined navigation and robot system 100) has sufficient information to register the acetabulum in the coordinate system (e.g., optical coordinate system) of the camera tracking system 200. In both of the above-noted example methods, the tracking system may be constantly monitoring and tracking the pose of the patient DRB 116 attached to the pelvis while also tracking the navigated instrument (e.g., stylus) such that the pose of the instrument can be tracked relative to the pose of the patient DRB, at least for purposes of registering the pelvis relative to the patient DRB 116 in the tracking coordinate system of the camera tracking system 200.

Some exemplary image-based examples include the use of pre-operative CT images. In one such exemplary image-based approach to patient registration, pre-operative CT and intra-operative fluoroscopy images are merged, the non-target surgical area is excluded, and the location of the target surgical area is registered based on the merged CT image and fluoroscopy image, as described in Patent Application No. 18/743,388 (Docket ROBOT.143.0002/ IDR-23-131). In another exemplary image-based approach to patient registration, pre-operative CT and intra-operative point cloud data acquired via a navigated instrument are merged, and the location of the target surgical area is registered based on the merged CT image and point cloud data, as described in Patent Application No. 18/743,615 (Docket ROBOT.143.0003 / IDR-23-151).

Other exemplary image-based registration approaches can be performed without first obtaining pre-operative CT images. In one such exemplary approach to patient registration, intra-operative fluoroscopy images are obtained, and an APP and FPP are identified. The FPP images and APP images are merged, excluding the non-target surgical area, and the location of the target surgical area is registered based on the merged APP and FPP fluoroscopy images, as described in Patent Application No. 18/743,647 (Docket ROBOT.143.0004 / IDR-23-149).

In a further exemplary image-based approach to patient registration, intra-operative fluoroscopy images are obtained, and intra-operative point cloud data is acquired using a navigated instrument. An FPP is identified in the intra-operative fluoroscopy images, and inputs from a navigated instrument about a location of the target surgical area are obtained. A set of landmarks relative to the identified FPP are verified using the inputs from the navigated instrument, and the location of the target surgical area is registered based on the identified FPP images and inputs from the navigated instrument, as described in Patent Application No. 18/743,685 (Docket ROBOT.143.0005/ IDR-24-008).

Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan.

### Robotic reaming workflow

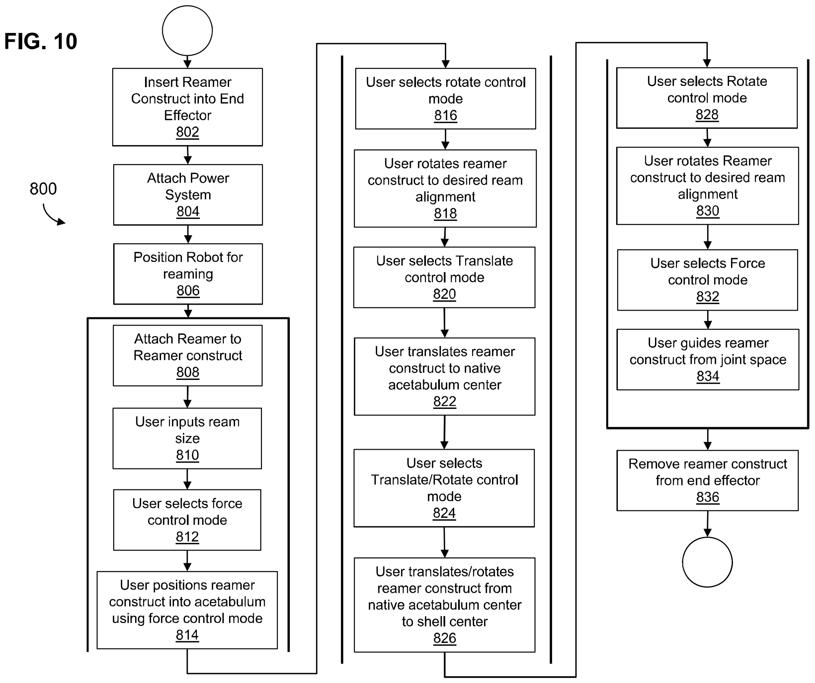

According to embodiments of the present disclosure, the surgical system 10, including surgical robot 100 as shown in, e.g., **FIGS. 1-4****,** may be used perform various workflows for robot-assisted surgical processes. With reference to **FIG. 10****,** one such workflow 800 may include preparing one or more bones such as, e.g., the acetabulum, for implant placement during THA surgery.

Preparation of the acetabulum may include reaming the acetabular cavity. The objective of the reaming workflow 800 may be to guide the reamer 124 into the patient's native acetabulum, and expand the diameter thereof to prepare the acetabular cavity for placement of an acetabular implant or "shell." In certain cases, the surgeon may use the reamer to modify the location of the native acetabulum center in accordance with a predefined surgical plan. The surgeon may perform the planned surgical procedure by holding the instrument, e.g., a navigated reamer construct with a reamer attached, and watching the procedure processes occur on the navigation system or the XR headset 150. The end effector 112 facilitates precise performance of the intended function of each instrument such as, e.g., the reamer 124 in workflow 800. In any robotic motion, a move enable switch must be engaged to allow the robotic movement.

Turning to workflow 800, at process 802, a sterile reamer construct 130 may be inserted into the end effector 112, e.g., into the guide tube 118. Alternatively, a sterile end effector integrated navigated reamer construct may be used. The reamer construct may be a navigated surgical instrument, and may include one or more instrument reference elements 170 attached to or formed on the instrument, as discussed elsewhere herein. The instrument reference element(s) 170 may be used to track the pose of the instrument, i.e. reamer, relative to, e.g., another fiducial and/or a defined coordinate system using a camera tracking system 200, a computer platform 400, and a display, which may include an XR headset 150 as described herein above.

At process 804, the power system may be connected to provide electrical power to robot 100 and other components of the system 10. The system may be powered on, and may perform a self-test. At process 806, components of the system including, e.g., the robot 100 may then be positioned proximate to the operating table, and stabilized on the floor. The robot 100 may then be draped to maintain sterility of the field.

Processes 808 through 834, described further herein below, are sub processes that collectively provide a process for preparing the patient's acetabular cavity for implantation of an acetabular prosthesis, or shell,, e.g., by reaming the acetabulum.

At process 808, an appropriately sized hemispherical acetabular reamer 124 may be coupled to the reamer construct 130 or driver. The reamer size, e.g., diameter, may be selected in accordance with a defined plan for the surgical procedure, and may be selected at least in part based on the anatomy of the patient.

At process 810, the surgeon may input or confirm the size of the selected reamer in the system 10. In certain embodiments, such input may be provided, e.g., via a touch screen or XR interaction using XR headset 150. The surgeon may then bring the robotic arm into position for the surgery by providing an input command, e.g., by pressing a foot pedal, or interacting with a touch screen or XR headset 150.

In a first stage of the robotically assisted reaming workflow 800, the reamer is inserted into the soft tissue of the patient to access the target surgical area. To perform the insertion, in process 812, the surgeon selects a force control, or haptic control mode, from a plurality of defined operational modes (or "operational control modes").

Each operational mode may be defined at least in part by the robotic arm movements, e.g., translation along the X-, Y-, and Z-axes, and rotation about each of these axes relative to defined coordinate system, that are either allowed or constrained while the particular operational mode is engaged. In this manner, the computer platform (e.g. computer platform 400) is adapted to selectively control the translation and orientation, e.g., the pose, of the navigated surgical instrument based on a defined operational mode to perform a process in the workflow as described herein. In certain embodiments, the operational mode may be surgeon-defined, e.g., by selection or actuation by the surgeon. This may be, e.g., via touch screen or XR interaction. In other embodiments, the operational mode may be defined by the system 10, e.g., as an automatic response to a predefined condition such a proximity of one particular navigated instrument, implant, or patient anatomical feature to another navigated instrument, implant, or patient anatomical feature. Each operational mode may be further defined by whether the mode is used in connection with navigational guidance, or whether the mode may be used without navigation guidance, e.g., manual manipulation of the instrument.

Referring back to process 812, in the force control mode, translation of the reamer along the X-, Y-, and Z-axes is permitted. Roll, yaw, and pitch of the reamer, i.e. rotation about each of the X-, Y-, and Z-axes are also permitted. Thus, in force control mode, the user may translate the instrument along any one or more of the X-, Y-, and Z-axes, and may rotate the instrument about any one or more of the X-, Y-, and Z-axes as shown in **FIG. 11** in order to gain access to the surgical target area, e.g., the acetabular cavity. The robotic arm may provide six active degrees of freedom (DOF). However, due to the axis of symmetry of the reamer construct 130 defined by the instrument rotation axis, only five DOF are required in order to fully define the position and orientation of the reamer construct in a defined coordinate system. Navigational guidance is not required in force control mode. The translation and orientation of the reamer 124 are controlled by the surgeon, through the direct or indirect application of force(s) and torque(s) on the end effector and/or the instrument. In certain embodiments, these force(s) and torque(s) may be directly applied to the end effector 112 or the reamer construct 130, while in others, the force(s) and torque(s) may correspond to force and torque control commands measured by the force and torque sensor integrated into a haptic control device and applied to the end effector.

At process 814, with the force control mode engaged, the user inserts the reamer 124 into the patient's native acetabulum. Due to the size constraints of the native acetabulum relative to the reamer 124, the center 132 of the reamer 124 will in many cases be located outward from the native center 134 of the acetabulum.

When proximity of the reamer 124 to the acetabulum is detected, e.g., using computer platform 400, the system may transition from the insertion stage to the alignment stage. In the alignment stage, the desired or defined ream trajectory is identified for the eventual reaming.

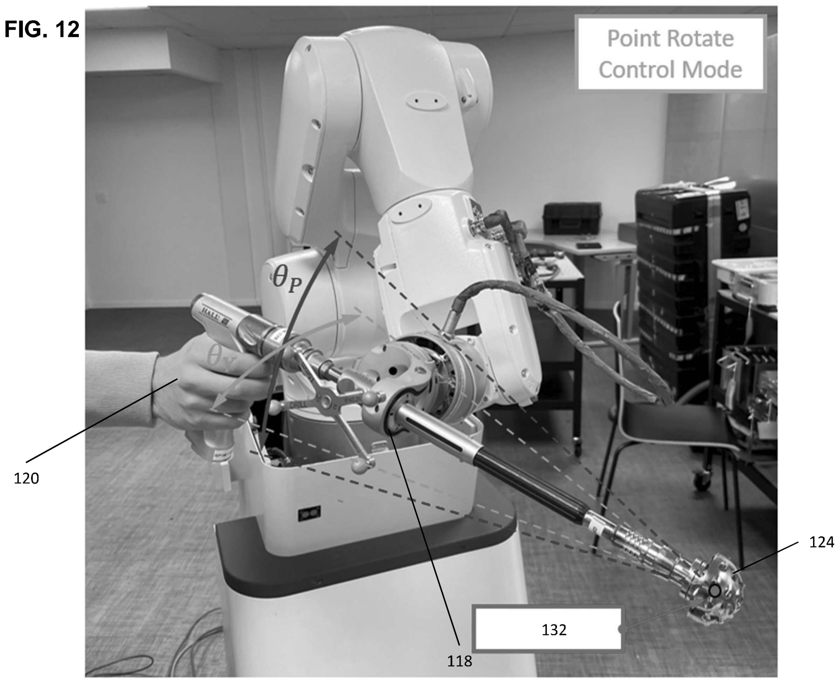

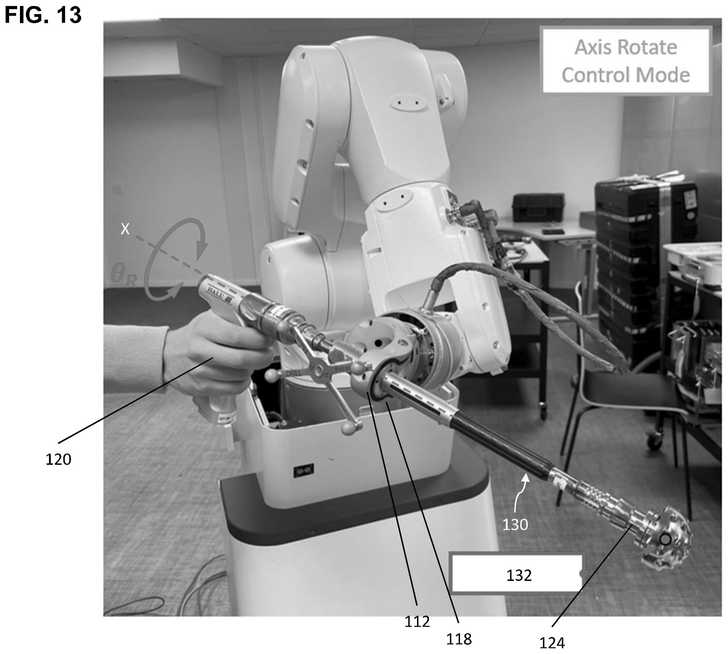

At process 816, to perform the aligning, the surgeon selects a rotate control mode from a plurality of defined operational modes (or "operational control modes"). The rotate control mode may be defined by constraint of translation of the reamer (e.g., the center point 132 of the reamer 124) along the X-, Y-, and Z-axes, while roll, yaw, and pitch, i.e. rotation of the reamer 124 about the X-, Y-, and Z-axes may be permitted. In particular, in a point rotate control mode, rotation about the Y- and Z- axes is permitted about a fixed and hard-coded point located at a center 132 of the reamer. In an axis rotate control mode, rotation is permitted about a trajectory defined by a rotational axis of the reamer (e.g., the X axis), and within a workspace of the robotic arm. In the example of **FIG. 12****,** pitch and yaw are permitted in point rotate control mode, while roll about the X-axis is permitted in axis rotate control mode, as illustrated in **FIG. 13****.** Rotation is in the control of the surgeon, and navigational guidance is not required while in rotate control modes.

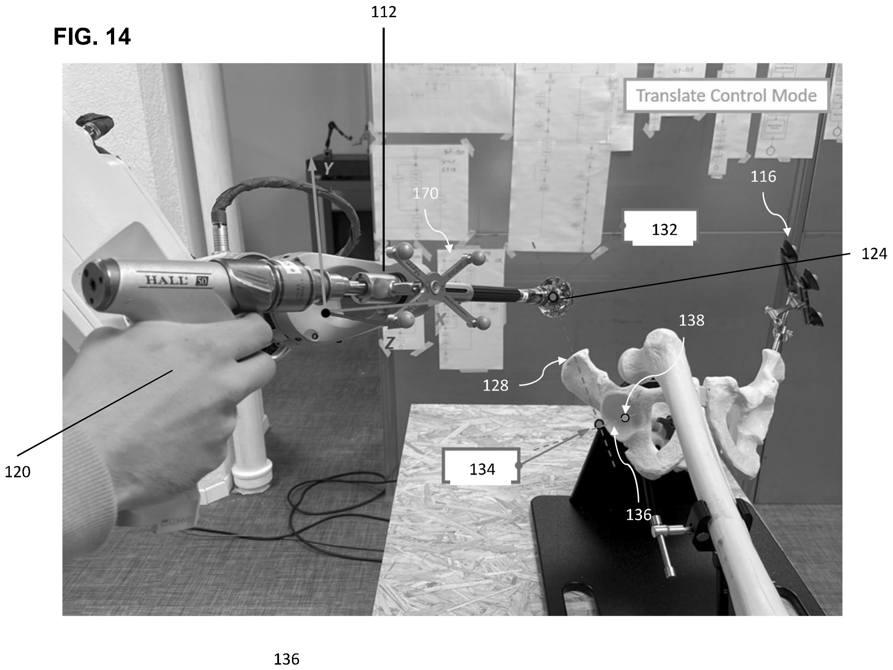

At process 818, the user actuates rotation of the reamer in rotate control mode to align the reamer construct 130 with the desired (i.e., defined or planned) ream trajectory 136 (illustrated in **FIG. 14**). In some embodiments, the computer platform 400 may be operative to identify the desired ream trajectory 136 and display the same, e.g., on a display or on XR headset 150 to guide the surgeon.

In process 820, the surgeon selects a translate control mode from the plurality of defined operational modes. The translate control mode is defined by constraint of rotation about the roll, yaw, and pitch axes X, Y, and Z, while permitting translation of the reamer along the X-, Y-, and Z-axes. Thus, translation may occur while maintaining the orientation of the instrument (e.g., reamer) previously achieved in process 818. Translation of the reamer is initiated by the user, and is assisted by navigational guidance provided by computer platform 400.

In process 822, the user actuates translation of the reamer in translate control mode to translate the reamer along a desired translation trajectory 128 (**FIG. 14**), without modifying the orientation of the reamer. Translation proceeds along the desired translation trajectory 128 until the center 132 of the reamer is colocalized with a native center of the acetabulum 134, and the reamer is aligned with the ream trajectory 136.

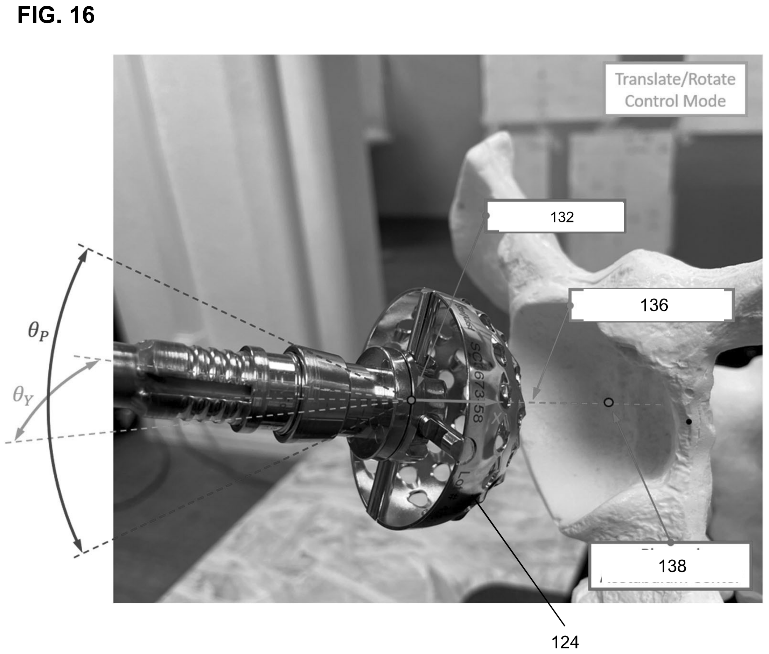

When completion of the alignment is detected, e.g., using computer platform 400, the system may transition from the alignment stage to the reaming stage. In the reaming stage, the acetabulum is reamed from the native center 134 thereof to the planned center of the acetabular prosthesis 138, along ream trajectory 136 (**FIGS. 14** and **16**).

At process 824, the surgeon selects a translate/rotate control mode from the plurality of defined operational modes. The translate/rotate control mode is defined by permission of the instrument to rotate about the fixed center point 132 of the reamer 124, and to translate along a defined ream trajectory 136, based on navigational guidance provide by computer platform 400, and upon actuation by the surgeon 120.

At process 826, when the surgeon is satisfied with the trajectory, rotation of the reamer 124 about the reamer center 132 may be actuated by the surgeon 120, for example by pulling a trigger on the reamer construct. Translation of the reamer along the defined ream trajectory 136 (**FIG. 16**), guided by computer platform 400, may be initiated by the surgeon by direct or indirect application of a force on the end effector as previously discussed. As the reamer 124 rotates about the reamer center 132 and translates along the defined ream trajectory 136 toward the planned center 138 of the shell, the bone is cut to prepare the acetabulum to receive the shell. Rotation of the reamer about the center point 132 (e.g. as shown in **FIG.** 12) during reaming may be useful to achieve a smooth prepared surface within the acetabular cavity, without leaving irregularities, e.g., relatively higher or lower points along the surface. When the center point 132 of the reamer 124 reaches the planned center point 138 of the shell, the computer platform is operative to stop the reaming process, so that the center 132 of the reamer 124 does not pass the planned center point 138 of the shell as it translates along the ream trajectory 136. In this manner, the system 10 realizes the surgical procedure planning, with the assistance of navigational guidance from computer platform 400, in order to localize the position and orientation of the reamer 124 with respect to the patient's anatomy.

In process 828, following the reaming, the surgeon selects the rotate control mode in a manner similar to process 816. As discussed above, the rotate control mode may be defined by constraint of translation of the reamer (e.g., the center point 132 of the reamer 124) along the X-, Y-, and Z-axes, while roll, yaw, and pitch, i.e. rotation of the reamer 124 about the X-, Y-, and Z-axes may be permitted.

In process 830, the surgeon rotates the reamer construct to a desired ream alignment to facilitate easy removal from the patient. In process 832, the surgeon selects force control mode in a manner similar to process 812. In process 834, the surgeon guides the reamer construct from the joint space in force control mode. As discussed above, navigational guidance is not required for processes 830 and 834, performed in rotate control and force control modes. In process 836, the surgeon removes the reamer construct from the end effector 112, concluding workflow 800.

In certain embodiments, processes 802 through 836 as described herein may be performed as a single stage reaming process, as illustrated in workflow 800 (**FIG. 10**). In other embodiments, one or more of processes, for example one or more of processes 812 through 834 may be performed iteratively as desired to achieve a satisfactorily prepared acetabular surface for the performance of processes 900 and 1000 as described herein. In other embodiments, certain phases of workflow 800 may be performed iteratively. For example, in one embodiment only the alignment stage, including processes 816 through 822, may be performed iteratively to achieve the desired alignment, followed by a single iteration of the reaming stage. In another embodiment, a single iteration of each of the insertion and alignment processes may be performed, followed by iterative performance of processes 824 and 826 in the reaming stage. Any combination of single iteration and multiple iteration may be used as needed to achieve the desired result.

### Robotic trialing workflow

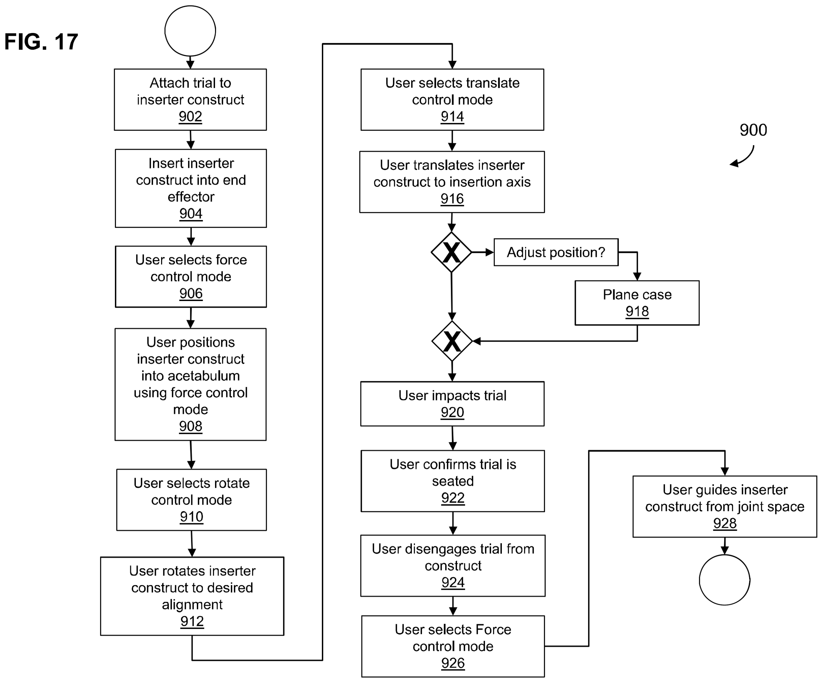

Trial placement, or trialing, is an optional workflow 900, illustrated in **FIG. 17****,** which may be performed in certain embodiments of the present disclosure, using the surgical system 10 including the surgical robot 100 (**FIGS. 1-4**) to assist the surgeon. Objectives of trialing may include assessing fitment of the shell in the acetabulum, which may be prepared according to workflow 800 as described herein, and assessing the interaction between femoral components, liner, and shell. Workflow 900 assists with ensuring that positioning of the shell in fact matches the pre-operative plan, and that the shell is fully seated in the prepared acetabulum.

Turning to workflow 900, a trial shell and an inserter construct are provided. Similar to reamer construct 130, the inserter construct may be a navigated surgical instrument, and may include one or more instrument reference elements 170 attached to or formed on the instrument, as discussed elsewhere herein. The instrument reference element(s) 170 may be used to track the pose of the instrument, i.e. the inserter, relative to, e.g., another fiducial and/or a defined coordinate system using a camera tracking system 200, a computer platform 400, and/or a display, which may include an XR headset 150 as described herein above. At process 902, the trial shell is attached to the inserter construct, and at process 904, the inserter construct is inserted into the end effector 112, e.g., into the guide tube 118. Alternatively, a sterile end effector integrated navigated inserter construct may be used.

At process 906, the user selects the force control mode of operation, in a manner similar to process 812 in workflow 800. Force control mode as used in workflow 900 is defined in the same manner set forth with respect to workflow 800. Thus, with the surgical robot 100 in force control mode, translation of the inserter along the X-, Y-, and Z-axes is permitted. Roll, yaw, and pitch of the inserter, i.e. rotation about each of the X-, Y-, and Z-axes are also permitted. Thus, in force control mode, the user may translate the instrument along any one or more of the X-, Y-, and Z-axes, and may rotate the instrument about any one or more of the X-, Y-, and Z-axes in order to gain access to the surgical area, and navigational guidance is not required. The translation and orientation of the inserter are controlled by the surgeon, through the direct or indirect application of force(s) and torque(s) on the end effector and/or the instrument.

At process 908, the user positions the inserter construct into the patient's acetabulum using force control mode. This movement brings the trial shell attached to the navigated inserter into the patient's previously prepared (e.g. via workflow 800) acetabulum.

At process 910, the user selects rotate control mode from the plurality of defined operational modes. The rotate control mode may be defined in the same manner as described above relative to process 816, e.g., translation of the inserter along the X-, Y-, and Z-axes are constrained, while roll, yaw, and pitch, i.e. rotation of the inserter about the X-, Y-, and Z-axes may be permitted. Rotation is in the control of the surgeon, and navigational guidance is not required while in rotate control modes.

At process 912, the user rotates the inserter construct to achieve the desired alignment and trajectory for insertion of the shell. In some embodiments, the computer platform 400 may be operative to identify the defined insertion trajectory and display the same, e.g., on a display or on XR headset 150 to guide the surgeon.

At process 914, the user selects translate control mode from the plurality of defined operational modes. Translate control mode may be defined in the same manner described above relative to process 820, e.g., by constraint of rotation about the roll, yaw, and pitch axes X, Y, and Z, and permission of translation of the inserter along the X-, Y-, and Z-axes. Thus, translation may occur while maintaining the orientation of the instrument (e.g., inserter) previously achieved in process 912. Translation of the inserter may be initiated by the user, and may be assisted by navigational guidance provided by computer platform 400.

At process 916, the user translates the inserter construct to the insertion axis. This process is analogous to process 822 of workflow 800 and the illustration of **FIG. 14****.**

At process 918, the user may optionally assess whether any adjustment is needed, e.g., in the planned trajectory or alignment of the inserter, and the adjustment may be made before proceeding to process 920. This assessment may be aided by visualization and/or navigational guidance from computer platform 400, which may be displayed, e.g., on a display or on XR headset 150.

At process 920, the user may impact the trial to seat it in the prepared acetabulum. At process 922, the surgeon may confirm that the trial is properly seated. For example, the trial may include a reference element that enables tracking thereof as described herein, and visualization to a user on a display or on XR headset 150. In some embodiments, the surgeon may, for example, review display or XR headset 150 to visually confirm whether the trial is properly seated. Other confirmation processes may be possible in other embodiments.

At process 924, the surgeon may disengage the trial from the inserter. At process 926, the surgeon may select force control mode, in a manner and as defined above relative to process 906. At process 928, the user guides the inserter from the joint space.

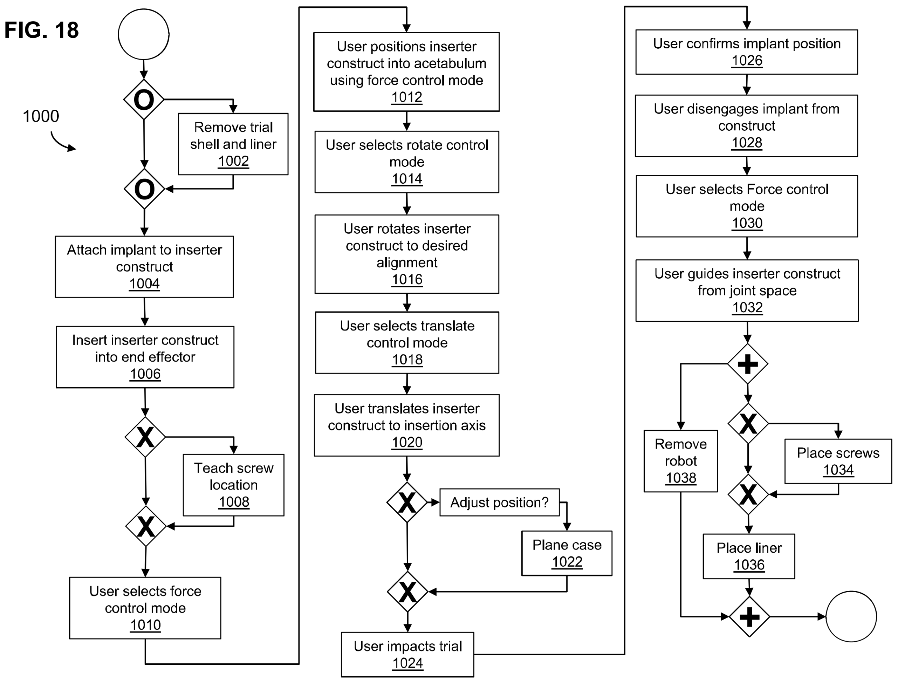

As noted, the trialing workflow 900 of **FIG. 17** is optional, and may be either performed or not performed following reaming workflow 800 (**FIG. 10**) and prior to implant placement workflow 1000 (**FIG. 18**) depending on a number of factors including, *inter alia,* the predefined surgical plan and/or judgment of the surgeon. In any case, if workflow 900 is performed, following its conclusion, the THA surgery may advance to workflow 1000.

### Implant placement workflows

**FIG. 18** illustrates a workflow 1000 for robot-assisted acetabular prosthesis (or "shell") placement in certain embodiments of the present disclosure, using the surgical system 10 including the surgical robot 100 (**FIGS. 1-4**) to assist the surgeon.

Turning to workflow 1000, in embodiments in which workflow 1000 optionally follows workflow 900, the surgeon begins at process 1002 by removing the any trial components from the navigated inserter, e.g., the trial shell and liner. In embodiments in which workflow 1000 immediately follows preparation of the acetabulum, e.g., via workflow 800, process 1002 may be omitted.

At process 1004, the user may attach or insert the shell or implant to the inserter construct, and at process 1006, the inserter construct may be inserted into the end effector 112.

At optional process 1008, in certain embodiments, the user may teach to the system 10 the location(s) of one or more fixation apertures, e.g., screw holes, in the shell using a registration workflow that may be, e.g., CT-based. In embodiments in which the locations of the fixation apertures are registered to the system 10 in process 1008, computer platform 400 may be adapted to provide navigational guidance enabling robot-assisted placement of the screws later in the workflow 1000, upon placement of the implant (see process 1034, discussed below).

At process 1010, the user selects the force control mode of operation, in a manner similar to processes 812 and 906 in workflows 800 and 900, respectively. Force control mode as used in workflow 1000 is defined in the same manner set forth with respect to workflows 800 and 900. Thus, with the surgical robot 100 in force control mode, translation of the inserter along the X-, Y-, and Z-axes is permitted. Roll, yaw, and pitch of the inserter, i.e. rotation about each of the X-, Y-, and Z-axes are also permitted. Thus, in force control mode, the user may translate the instrument along any one or more of the X-, Y-, and Z-axes, and may rotate the instrument about any one or more of the X-, Y-, and Z-axes in order to gain access to the surgical area, and navigational guidance is not required. The translation and orientation of the inserter are controlled by the surgeon, through the direct or indirect application of force(s) and torque(s) on the end effector and/or the instrument.

At process 1012, the user positions the inserter construct into the patient's acetabulum using force control mode. This movement brings the shell implant attached to the navigated insert into the patient's previously prepared (e.g. via workflow 800) acetabulum.

At process 1014, the user selects rotate control mode from the plurality of defined operational modes. The rotate control mode may be defined in the same manner as described above relative to processes 816 and 910, e.g., translation of the inserter along the X-, Y-, and Z-axes are constrained, while roll, yaw, and pitch, i.e. rotation of the inserter about the X-, Y-, and Z-axes may be permitted. Rotation is in the control of the surgeon, and navigational guidance is not required while in rotate control modes.

At process 1016, the user rotates the inserter construct to achieve the desired alignment and trajectory for insertion of the shell. In some embodiments, the computer platform 400 may be operative to identify the defined insertion trajectory and display the same, e.g., on a display or on XR headset 150 to guide the surgeon.

At process 1018, the user selects translate control mode from the plurality of defined operational modes. Translate control mode may be defined in the same manner described above relative to processes 820 and 914, e.g., by constraint of rotation about the roll, yaw, and pitch axes X, Y, and Z, and permission of translation of the inserter along the X-, Y-, and Z-axes. Thus, translation may occur while maintaining the orientation of the instrument (e.g., inserter) previously achieved in process 1016. Translation of the inserter may be initiated by the user, and may be assisted by navigational guidance provided by computer platform 400.

At process 1020, the user translates the inserter construct to the insertion axis. This process is analogous to processes 822 and 916 of workflows 800 and 900, respectively, and the illustration of **FIG. 14****.**

At process 1022, the user may optionally assess whether any adjustment is needed, e.g., in the planned trajectory or alignment of the inserter, and the adjustment may be made before proceeding to process 1024. This assessment may be aided by visualization and/or navigational guidance from computer platform 400, which may be displayed, e.g., on a display or on XR headset 150.

Upon a determination that the trajectory and alignment of the inserter, and the planned location of the implant are satisfactory, at process 1024, the user may impact the shell to seat it in the prepared acetabulum. At process 1026, the surgeon may confirm that the implant is properly positioned and seated, in a manner similar to that described herein relative to process 922 in workflow 900.

At process 1028, the surgeon may disengage the shell from the inserter. At process 1030, the surgeon may select force control mode, in a manner and as defined above relative to process 1010. At process 1032, the user guides the inserter from the joint space.

Following removal of the inserter at process 1032, the user may optionally place one or more screw(s) at process 1034 to augment fixation of the shell in the acetabulum. As discussed above relative to process 1008, in certain embodiments in which the fixation apertures in the shell have previously been registered to the system 10, the computer platform 400 may be operative to provide navigational guidance for robotic placement of the screws in the fixation apertures in the shell. In other embodiments, in which the fixation apertures in the shell were not previously been registered to the system 10 (i.e., optional process 1008 is omitted), then screws may be placed without the benefit of navigational guidance and/or robotic assistance. In still other embodiments, as screw placement 1034 is an optional process, it may be omitted entirely.

Following removal of the inserter at process 1032, and also following placement of screws at process 1034 (if performed), a liner may be inserted into the shell at process 1036.

Also following removal of the inserter at process 1032, at process 1038, the surgical robot 10 may be removed from the surgical field. In certain embodiments, process 1038 may occur in parallel with one or more of processes 1034 and/or 1036.

Workflows 800, 900, and 1000 for robotically-assisted acetabulum preparation and prosthesis placement provide a number of advantages compared to previous methods. For example, workflow 800, as assisted by system 10 including surgical robot 100, provides additional accuracy and force assistance when preparing the acetabulum compared to prior methods. Trial and prosthesis placement according to workflows 900 and 1000 provide improved accuracy and alignment. Certain embodiments also provide beneficial confirmation of screw location prior to drilling, when used in combination with a CT-based registration workflow.

### Attraction Volume-Based Control

Particular implementations provide a surgical robot with a controller programmed to use an attraction volume based control protocol in a THA procedure. In certain cases, the surgical robot controller is configured to detect a position of a surgical instrument in one or more surgical volumes, and provide guidance (e.g., an attraction force) to movement of the surgical instrument in a direction toward the attraction volume. In various implementations, the attraction volume(s) are defined in part by a point and an axis extending from the point. Preferably each attraction volume can be codified in program code and defined by coordinates (e.g., three dimensional coordinates in a surgical space) that are associated with a tracked position of the surgical instrument 1200. Each attraction volume is defined in part by a point (e.g., acetabulum center 1220) and an axis (A) extending from the point 1220. In particular cases, the point 1220 and the axis (A) define a centerline (CL) of each of the attraction volumes. In certain implementations, at least one of the attraction volumes has an approximately conical shape, e.g., having a larger radius at a distance farther from the point 1220 than at a distance closer to the point 1220. In this case the axis (A) is the vertical axis of the cone, i.e. the axis that is perpendicular to the base of the cone and passes through the point. The point is in this case is the vertex of the cone. In some cases, the conical shaped attraction volume(s) are truncated by a plane (P) that intersects the point 1220, preferably the plate (P) is perpendicular to the axis (A). In further version each one of the attraction volumes has an approximately tubular shape, such that the radius of the volume 1250 is approximately consistent along the axis (A) and the axis is the longitudinal axis of the tubular shape.

In particular examples, when the surgical instrument (e.g., instrument tip) is detected by the navigation system within the a first (e.g., "Low Attraction") volume, the surgical robot controller (e.g., via robotic arm) provides (relatively) low assistance by generating forces and torques that are added to those applied by the user. This assistance helps guide the surgical instrument (e.g., instrument tip) onto the planned acetabulum trajectory and aligns the surgical instrument axis with the planned trajectory. In some examples, in the "Low Attraction" volume, the robotic forces and torques are of the same order of magnitude as the user's applied forces and torques but remain within magnitudes that the user can override if needed. As such, the user may feel a low attraction toward the planned acetabulum trajectory but can still move the surgical instrument center out of the "Low Attraction" volume by applying appropriate forces and torques in the opposite direction and intensity to the robotic assistance.

Proximity attraction-based control can also be performed within a second (or third, or additional) volume (e.g., a "High Attraction" volume or "higher attraction" volume). In these cases, when the surgical instrument (e.g., instrument tip) leaves the "Low Attraction" volume and is detected inside the "High Attraction" volume(s) by the navigation system, the robot controller (e.g., robotic arm) generates a high assistance in the form of a torsor of forces and torques with intensity higher than torsor of forces and torques applied by the user, in order to bring the instrument center (instrument tip) onto the planned acetabulum trajectory and align the instrument axis to the planned acetabulum trajectory. A torsor of force in physics is defined as the vector containing the intensity of the three forces applied along the three orthogonal directions of the 3D space, and the intensity of the torques applied around the same three directions. In certain of these cases, the user will feel a high intensity attraction of the instrument toward the planned acetabulum trajectory.

In still further cases, the robot controller is configured to operate in a fixed mode once the surgical instrument aligns with the planned acetabulum trajectory, e.g., once the surgical instrument aligns with the point and/or axis of the trajectory. In certain such cases, the robot controller does not enable movement of the surgical instrument in at least one degree of freedom (DoF) once it is aligned with the planned acetabulum trajectory. In this sense, the robot controller fixes the surgical instrument on the planned acetabulum trajectory until a user (or another command control) overrides the operating mode.

As described in US 10,058,394 ("Robot Arm and Methods of Use," issued August 28, 2018 and incorporated by reference in its entirety), attraction forces and/or resistance to movement of the surgical instrument can be controlled via a robot arm, e.g., at the end effector. More particularly, a controller (or multiple controllers such as one or more microcontrollers) can be programmed to control one or more motors operable to move the robot arm and/or end effector to provide guidance via attraction forces and/or resistance to the user of the surgical instrument during the THA procedure. Feedback from one or more tracking systems such as those relying on the reference element(s) 170 described herein can enable precise tracking of the location of the surgical instrument in the space, allowing for real-time surgical guidance via haptics.

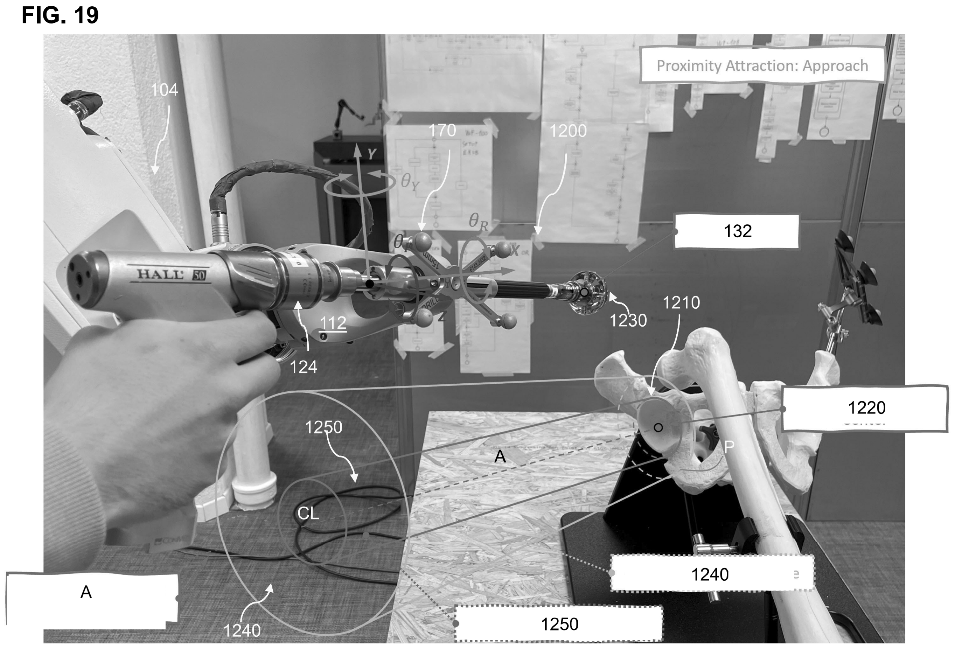

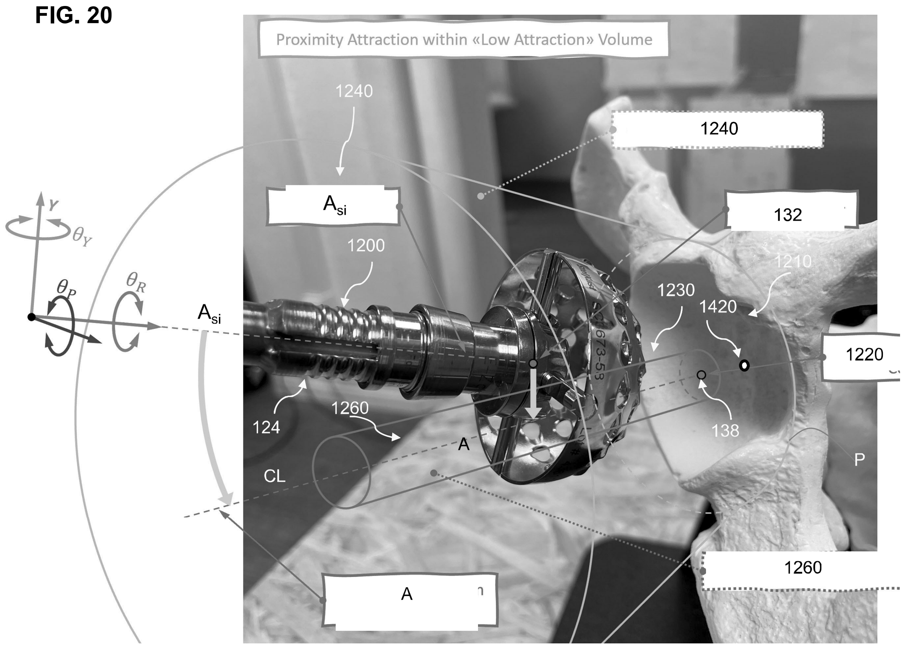

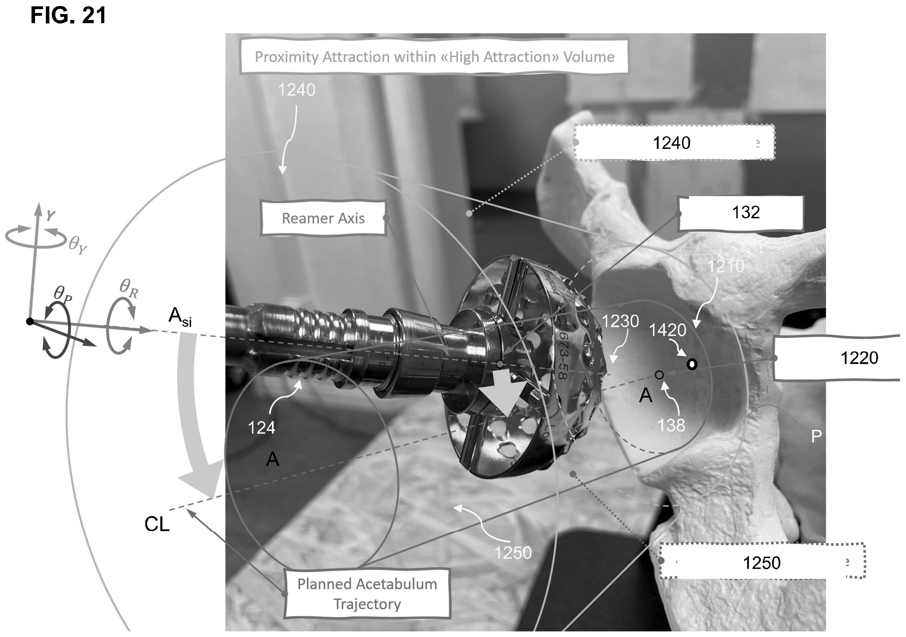

**FIG. 19** shows an example of a surgical instrument 1200 and an acetabulum region 1210 including a planned acetabulum center 1220 according to various implementations. In some cases, the surgical instrument 1200 includes a reamer, an impactor, or another surgical instrument described herein. In some cases, the surgical instrument 1200 is similar to reamer 124 described herein, e.g., including a center 132. The surgical instrument 1200 is coupled to a robot arm 104, e.g., via an end effector 112. **FIG. 20** and **FIG. 21** show close-up perspective views of the distal end (or, tip) 1230 of the instrument 1200, in this example, the distal end 1230 of a reamer 124 for reaming the acetabulum region 1210. As described herein, the teachings of various embodiments can apply to distinct types of surgical instrument 1200, e.g., the distal end of an impactor, reamer, etc.

**FIGS. 19-21** also illustrate distinct attraction volumes 1240, 1250, 1260 for use in guiding the surgical instrument 1200 according to distinct implementations herein. Two attraction volumes 1240, 1250 are shown in some examples, however, one or more attraction volumes can be used to perform beneficial control functions described herein. For example, a single attraction volume can be used to control the surgical instrument 1200 during a THA procedure, or two, three, four, or more attraction volumes can be used to control the surgical instrument 1200 during a THA procedure. **FIG. 20** illustrates an example configuration including additional attraction volume 1260 within attraction volume 1240, that has a smaller radius than attraction volume 1250 illustrated in **FIG. 19** and **FIG. 21****.** In certain implementations, attraction volume 1260 is used in conjunction with both attraction volume 1240 and attraction volume 1250. For the purposes of efficient explanation, attraction volume 1240 can be considered a first attraction volume, while attraction volumes 1250 and 1260 can be considered second attraction volumes, or second and third attraction volumes, respectively.

It is understood that the attraction volumes 1240, 1250, 1260 are defined by a set of coordinates and/or a perimeter of coordinates with a center point, axis, or other reference point(s) that relate to a target location for the THA procedure. For example, the attraction volumes 1240, 1250, 1260 can be defined based on the planned acetabulum center 1220 (e.g., a center of the implant placed in the acetabulum region 1210). The attraction volumes 1240, 1250, 1260 can be codified in program code (e.g., at navigation controller 404 or a distinct processor at the computer platform 400, **FIG. 4**), and defined by coordinates (e.g., three dimensional coordinates in a surgical space) that are associated with a tracked position of the surgical instrument 1200 (e.g., as indicated by reference element 170, e.g., **FIG. 11****,** **FIG. 14****,** **FIG. 19**). That is, the attraction volumes 1240, 1250, 1260 will not necessarily be visible to the operator (e.g., surgeon) during the THA procedure, but will provide guidance via control functions at the robot arm 104. In particular implementations, the attraction volumes 1240, 1250, 1260 provide haptic feedback (or, guidance) to the user during the THA procedure. In additional implementations, for example, where XR headset 150 **(****FIG. 1**) is used, the attraction volumes 1240, 1250, 1260 can be overlayed in a display that is visible to the user(s) during the THA procedure. In such cases, a perimeter, center line, and/or point(s) defining the attraction volume(s) 1240, 1250, 1260 can be visibly displayed to the user(s) during the THA procedure to aid in guiding the surgical instrument 1200 to a target location.

As described herein, the attraction volumes 1240, 1250, 1260 are defined in part by a point (e.g., acetabulum center 1220) and an axis (A) extending from the point 1220. In particular cases, the point 1220 and the axis (A) define a centerline (CL) of both the first attraction volume 1240 and the second attraction volume(s) 1250 and/or 1260. In certain implementations, at least one of the attraction volumes has an approximately conical shape, e.g., having a larger radius at a distance farther from the point 1220 than at a distance closer to the point 1220. In this case the axis (A) is the vertical axis of the cone, i.e. the axis that is perpendicular to the base of the cone and passes through the point. The point is in this case is the vertex of the cone. In some cases, the conical shaped attraction volume(s) are truncated by a plane (P) that intersects the point 1220, preferably the plate (P) is perpendicular to the axis (A). In additional implementations, e.g., as shown in attraction volume 1250 in **FIG. 19****,** the volume may have an approximately tubular shape, such that the radius of the volume 1250 is approximately consistent along the axis (A) and the axis is the longitudinal axis of the tubular shape.

In certain implementations, the navigation controller 404 (or, other controller and/or microcontroller at computer platform 400) is configured, during a THA procedure, to: i) detect a position of the surgical instrument 1200 in at least one of the attraction volumes 1240, 1250, 1260, and ii) while in the attraction volume(s) 1240, 1250, 1260, provide an attraction force to guide movement of the surgical instrument 1200 in a direction toward the attraction volume 1240, 1250, 1260. In particular cases, the controller 404 provides guidance (or, an attraction force) to movement of the surgical instrument 1200 in directions toward the planned trajectory of the surgical instrument 1200 for the THA procedure. For example, the controller 404 can provide an attraction force to move the surgical instrument 1200 in direction(s) toward the axis (A) and/or the point (e.g., 1220) that define the planned trajectory. In certain examples, if the user does not apply any force on the surgical instrument 1200 while that instrument 1200 is within the attraction volume(s) 1240, 1250, 1260, the navigation controller 404 provides an attraction force sufficient to move the surgical instrument 1200 toward the planned trajectory (e.g., toward axis (A) and/or point 1220). In a particular example, the controller 404 is configured to enable movement of the surgical instrument 1200 (e.g., via end effector 112 and/or robot arm 104) in multiple directions, but provide greater assistance to movement in directions toward the planned trajectory (e.g., axis (A) and/or point 1220). In various implementations, the directional assistance is based in part on the shape and size of the attraction volume(s) 1240, 1250, 1260, etc. For example, the controller 404 can be configured to provide assistance to movement in directions that are radially inward toward the axis (A) and/or axially inward toward the acetabulum center 1220. That is, as the surgical instrument 1200 enters the attraction volume(s) 1240, 1250, 1260, the controller 404 actuates motors or other limiters in the robot arm 104 and/or end effector 112 to provide attraction forces (e.g., haptic guidance) in a radially inward direction (toward axis (A)), and/or to guide movement in an axial direction toward the acetabulum center 1220 along axis (A). It is understood that should the surgical instrument 1200 move through an attraction volume 1240, 1250, 1260 from one side of axis (A) to the other side of axis (A), the controller 404 will respond by adjusting assistance back in a direction that guides the surgical instrument 1200 toward the axis (A).

In particular implementations, distinct attraction volumes can have distinct associated attraction forces. For example, a first attraction volume such as attraction volume 1240 can have a lesser attraction force than a second attraction volume such as attraction volume 1250 (or 1260). As noted herein, in certain cases, the second (or third) attraction volumes 1250, 1260, etc. can be sub-volumes of the first attraction volume 1240. In this sense, as the surgical instrument 1200 enters the first attraction volume 1240, the user is met with an attraction force that guides movement in directions toward the planned trajectory (e.g., radially toward axis (A) and/or axially toward point 1220). In particular cases, the attraction force within a given attraction volume is approximately consistent, such that the user feels approximately the same amount of attraction force while within the attraction volume regardless of a distance the surgical instrument 1200 is located from the axis (A) and/or point 1220). In other cases, attraction force within a given attraction volume is progressive, such that the user feels greater attraction force to movement toward the planned trajectory (e.g., toward axis (A) and/or point 1220) as the surgical instrument 1200 moves closer to the planned trajectory. In various implementations where second (or third) attraction volumes 1250, 1260 are sub-volumes of the first attraction volume 1240, the user feels greater attraction force to movement toward the planned trajectory (e.g., toward axis (A) and/or point 1220) as the surgical instrument 1200 enters the second (or third) attraction volume(s) 1250, 1260. In these cases, the user (or, operator) must exert greater force to remove the surgical instrument 200 from the second (or, third) attraction volume(s) 1250, 1260 than the first attraction volume 1240. As noted herein, any of the attraction volumes can have an approximately consistent attraction forces, or a progressive attraction forces.

According to certain embodiments, the controller (e.g., navigation controller 404 or other controller and/or microcontroller at computer platform 400) is further programmed to progressively guide a tip (e.g., distal end) 1230 of the surgical instrument 1200 to the point 1220, and an axis (Aₛᵢ) of the surgical instrument 1200 to the axis (A) of the attraction volume 1240. In various embodiments, progressively guiding the surgical instrument 1200 includes providing haptic feedback to guide the tip 1230 of the surgical instrument 1200 to the point 1220, and subsequently providing haptic feedback to guide the axis (Aₛᵢ) of the surgical instrument 1200 to the axis (A) of the attraction volume 1240. As noted herein, the haptic feedback can include providing greater attraction force to movement of the surgical instrument 1200 in directions toward the point 1220 and the axis (A) of the attraction volume 1240. In some cases, progressively guiding the tip 1230 and then the axis (Aₛᵢ) of the surgical instrument 1200 aids in efficiently drawing the surgical instrument to the planned trajectory of the surgical instrument. For example, it may be easier for the operator to first align two points, e.g., the tip 1230 with the point 1220, and subsequently align the axes (e.g., A and Aₛᵢ), each of which consists of a plurality of points. The controller 404 can be configured to provide instructions (e.g., via display 110 and/or interface 440) and/or guided attraction to first align the tip 1230 with the point 1220, and subsequently align the axes (e.g., A and Aₛᵢ). As also noted herein, the axis (A) of the attraction volume 1240, 1250, 1260 is aligned with the planned trajectory of the surgical instrument 1200 for the THA procedure.

In additional implementations, providing haptic feedback (e.g., navigation controller 404 or other controller and/or microcontroller at computer platform 400) can include providing other haptically detectable outputs to the user that aid in guiding the surgical instrument 1200 toward the planned trajectory. For example, additional or alternative haptic feedback can include causing the end effector 112 and/or robot arm 104 to vibrate in response to user movement of the surgical instrument 1200 in a direction away from the planned trajectory. In these cases, while the surgical instrument 1200 is in an attraction volume 1240, 1250, etc., the controller initiates vibration of the end effector 112 and/or robot arm 104 in response to the user moving the surgical instrument away from axis (A) and/or point 1220. This vibrational feedback can be in addition to, or an alternative to, attractive forces to movement via the motorized and/or limiter-based approaches described herein. In various additional embodiments, auditory feedback may be provided to the user in addition to, or as an alternative to the haptic feedback described herein. For example, XR headset 150 may play out auditory feedback to the user, which may include one or more patterns of tones or verbal feedback representative of particular movements of the surgical instrument 1200.

As noted herein, in certain implementations, the controller 404 (or, other controller and/or microcontroller at computer platform 400) is configured to operate in multiple modes during the THA procedure. In some cases, the controller 404 is configured to automatically switch between operating modes in response to detecting that the surgical instrument 1200 is aligned with the planned trajectory of the surgical instrument for the THA procedure (e.g., point 1220 and axis A). In particular embodiments, the automatic switching is performed without requiring additional user interaction (e.g., via a command interface 440 or other interface such as a GUI at display 110).

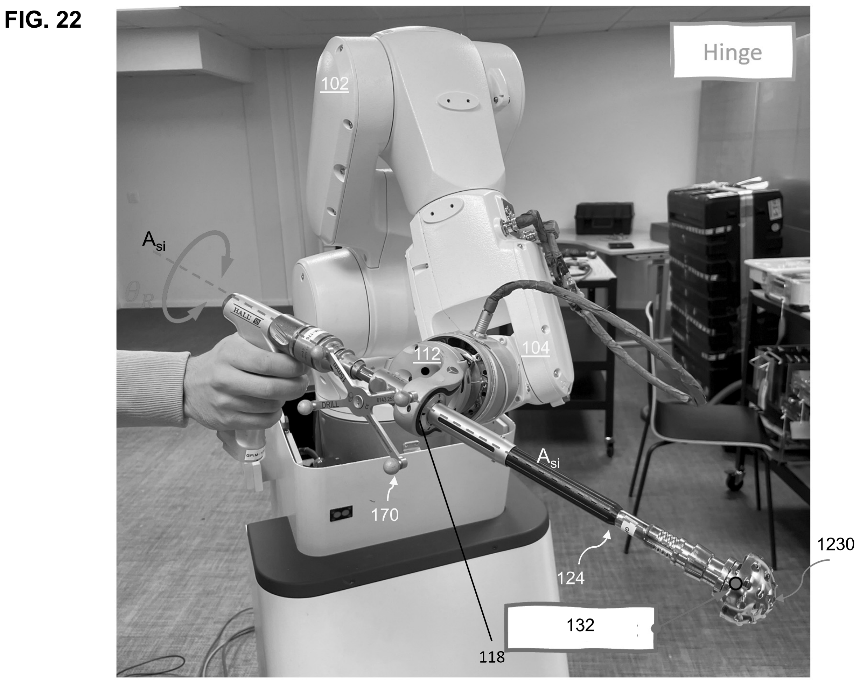

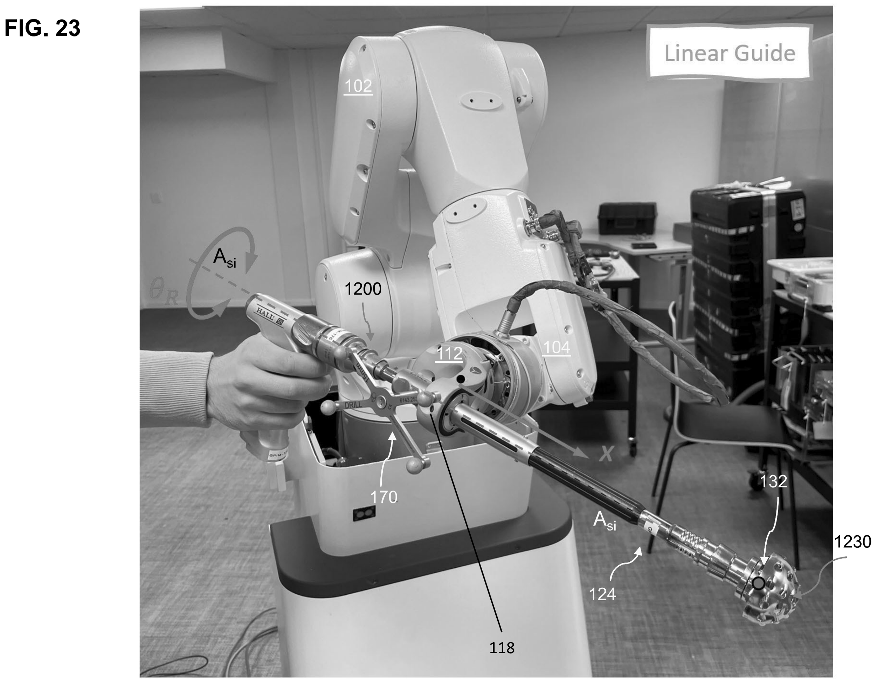

In particular embodiments, operating modes include a free mode, a constrained mode, etc. In particular embodiments, the constrained mode includes a hinge sub-mode, a fixed sub-mode, etc. **FIG. 22** shows an example of a hinge sub-mode (or, a hinge haptic control mode), and **FIG. 23** illustrates a linear guide mode. According to certain embodiments, the hinge haptic control mode depicted in **FIG. 22** limits degrees of freedom of the surgical instrument 1200 to permit rotation around the axis (Aₛᵢ) thereof. In these implementations, the tip 1230 position is fixed (e.g., in alignment with point 1220), and the axis (Aₛᵢ) of the surgical instrument 1200 is fixed to the axis (A), such that the surgical instrument 1200 is configured to rotate around the axis (A) as indicated by θ_{R}.

According to certain embodiments, another operating mode includes a linear guide haptic control mode (**FIG. 23**) that limits degrees of freedom of the surgical instrument 1200 to permit translation along the axis (A). In these cases, once the axis (Aₛᵢ) of the surgical instrument 1200 is aligned with the axis (A), the user can translate the surgical instrument 1200 along the axis (A), as well as rotate about the axis (A) as indicated by θ_{R}.

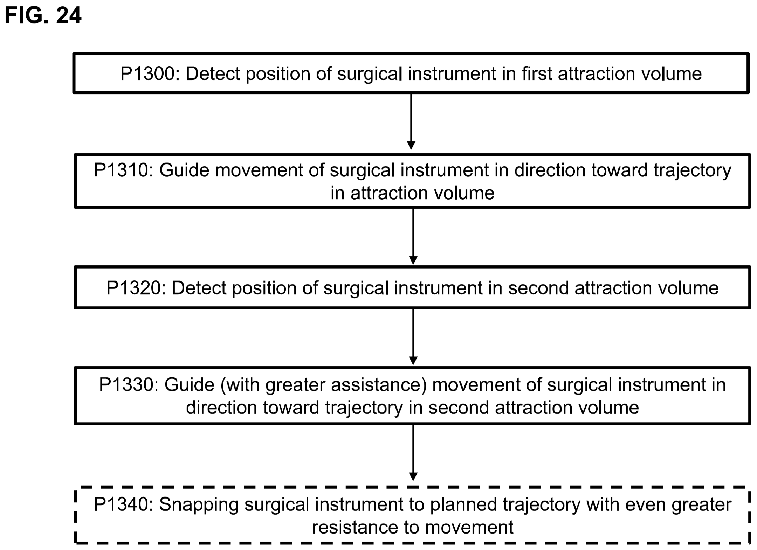

In particular implementations, as shown in the process flow diagram in **FIG. 24****,** the controller 404 can be configured to control a portion of the THA procedure using multiple attraction volumes 1240, 1250, 1260 by:

P1300: detecting the position of the surgical instrument 1200 in a first attraction volume 1240 during a THA procedure;

P1310: providing attraction forces to movement of the surgical instrument 1200 in a direction toward the first attraction volume 1240 (e.g., radially toward axis (A) and/or linearly toward point 1220);

P1320: detecting the position of the surgical instrument 1200 in a second attraction volume 1250, 1260 within the first attraction volume 1240; and

P1330: providing greater attraction forces to movement of the surgical instrument 1200 in a direction toward the second attraction volume 1250, 1260 (e.g., radially toward axis (A) and/or linearly toward point 1220). Optional implementations (indicated in phantom) can also include:

P1340: snapping the surgical instrument 1200 to the planned trajectory (e.g., axis A and point 1220) of the surgical instrument 1200 for the THA procedure, for example, with the relative greatest attraction force. In these cases, snapping the surgical instrument 1200 to the planned trajectory can be performed with higher attraction force than in P1330, and in certain cases, includes providing sufficient attraction force that a user would require deliberate, high-force actuation to move the surgical instrument from the planned trajectory. In some cases, process 1340 is performed when the position of the surgical instrument is within a threshold distance or threshold percentage deviation from the planned trajectory, for example, within X centimeters of axis (A) and/or point 1220 and/or within Y percent deviation from axis (A) and/or point 1220. In particular implementations, snapping can be enabled or disabled via settings for the THA procedure.

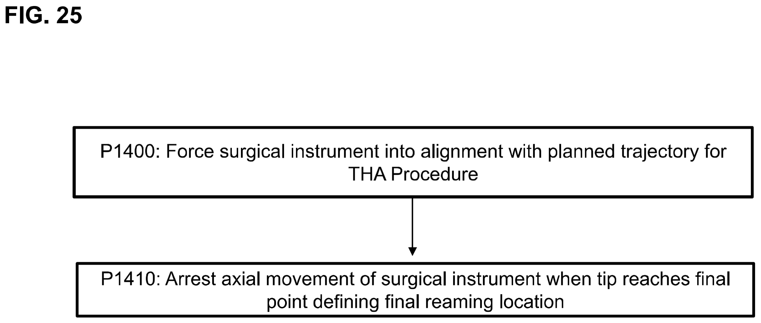

According to additional particular implementations, with reference to the flow diagram of **FIG. 25** and the schematic depiction in **FIG. 20****,** the controller 404 is configured to control a reaming portion of a THA procedure by:

P1400: forcing (e.g., snapping) the surgical instrument 1200 into alignment with the planned trajectory for the THA procedure (e.g., axis A and point 1220) such that a tip 1230 of the surgical instrument 1200 is coincident with the point 1220 (planned acetabulum center). In these cases, the planned acetabulum center 1220 defines both the planned center point for the acetabulum 138, and an initial reaming location for reaming the acetabulum cavity; and

P1410: arresting axial movement (e.g., along axis A) of the surgical instrument 1200 when the tip 1230 reaches a final point 1420 that defines a final reaming location. The final reaming location is understood to define a position at which the surgical instrument 1200 is located when the reaming process is complete. In various implementations, as shown in **FIG. 20****,** the final reaming location 1420 is distal of the initial reaming location 1220. In particular cases, the final reaming location (or final point) 1420 relates to the acetabulum center point 138, as well as a size of the implant. In other terms, the final reaming location (or point) 1420 is predetermined (or preselected) based on the planned acetabulum center point / implant size. In particular examples, the final reaming location 1420 is axially offset from the acetabulum center point 138 by a radius of the acetabulum implant or shell.

In some cases, after forcing the surgical instrument 1200 into alignment with the planned trajectory (e.g., axis A and point 1220), the surgical instrument 1200 is free to rotate about the axis (A) or to pivot about the point 1220. In further cases, the surgical instrument 1200 is free to rotate about the axis (A) or pivot about the point 1420 after axial movement has been arrested. In some examples, the controller 404 arrests movement of the surgical instrument 1200 after arriving at the final destination (point 1420), such that no more movement is permitted, even if the user actuates the instrument 1200. In these cases, the reamer may be free to spin (e.g., via actuation by the user), but the instrument 1200 is fixed so it will not enable further reaming depth along axis (A). As noted in example implementations herein, in certain examples, the controller 404 snaps the center point 1220 of the planned acetabulum to the end (e.g., tip) 1230 of the surgical instrument (e.g., reamer) 1200, defining an initial reaming location. At the initial reaming location 1220, the controller 404 enables the user to pivot/rotate the instrument around the point 1220, and allows for reaming, which removes tissue linearly; and after reaming, arrives at the second center point (final center point) 1420, where the controller arrests axial movement of the surgical instrument (e.g., still enabling rotation/pivoting about point 1420).

According to certain embodiments, as noted herein, the position of the surgical instrument 1200 can be tracked via one or more reference elements 170, and a display 110 and/or user interface such as 440 can enable a user (e.g., surgeon, technician, assistant, etc.) to monitor the position of the surgical instrument 1200 in the surgical space during the THA procedure. The surgical robot can include a navigation system (controller 404) for tracking the reference element(s) 170 and providing inputs to the controller 404 for performing functions described herein, as well as providing feedback via the display 110 and/or user interface 440 to the user.

In contrast to conventional THA procedures, the approaches and systems disclosed herein can enable greater precision in locating surgical instruments in multiple phases of surgery. Further, the approaches and systems disclosed herein can increase the efficiency of THA procedures by effectively guiding surgical instruments to a planned trajectory. The disclosed approaches and systems are also configured to limit user error in reaming and/or implant placement, by guiding movement and/or actuation of a surgical instrument in a manner that deviates from the planned trajectory.

### Post-operative Assessment Example

In a manner similar to that of a pre-operative patient assessment, the system 10 may be used by the surgeon to perform a post-operative patient assessment, to assess the quality of the THA surgery and the benefits to the patient. The workflow can include a patient mobility evaluation, e.g., with data collected from physical exercises and sensor feedback (e.g., attached to the leg), and which can be compared to clinical surveys to confirm the patient's health status. All gathered post-operative information can be automatically compared by the system to the pre-operative information.

### Further Definitions and Embodiments

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected," "coupled," "responsive," or variants thereof to another element, it can be directly connected, coupled, or responsive to the other element or intervening elements may be present. In contrast, when an element is referred to as being "directly connected," "directly coupled," "directly responsive," or variants thereof to another element, there are no intervening elements present. Like numbers refer to like elements throughout. Furthermore, "coupled," "connected," "responsive," or variants thereof as used herein may include wirelessly coupled, connected, or responsive. As used herein, the singular forms "a," "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise," "comprising," "comprises," "include," "including," "includes," "have," "has," "having," or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.," which derives from the Latin phrase "exempli gratia," may be used to introduce or specify a general example or examples of a previously mentioned item, and is not intended to be limiting of such item. The common abbreviation "i.e.," which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

The invention could be inter alia defined by the following examples:
1. A surgical robot for use in a total hip arthroplasty (THA) procedure, the robot comprising: a robot arm for holding a surgical instrument; and a controller coupled with the robot arm, the controller programmed to: detect a position of the surgical instrument in at least one attraction volume corresponding with a planned trajectory of the surgical instrument for the THA procedure, the at least one attraction volume defined in part by a point and an axis extending from the point; and while the surgical instrument is in the at least one attraction volume during the THA procedure, provide assistance to movement of the surgical instrument in a direction toward the at least one attraction volume.
2. The surgical robot of Example 1, wherein the at least one attraction volume includes multiple attraction volumes having distinct associated attraction forces.
3. The surgical robot of Example 2, wherein a first attraction volume has a lesser attraction force than a second attraction volume, and wherein the second attraction volume is a sub-volume of the first attraction volume.
4. The surgical robot of Example 3, wherein the point and the axis define a centerline of both the first attraction volume and the second attraction volume.
5. The surgical robot of Example 3, wherein an operator must exert greater force to remove the surgical instrument from the second attraction volume than the first attraction volume, and wherein the controller is configured to fix the surgical instrument in alignment with the planned trajectory in at least one operating mode.
6. The surgical robot of Example 1, wherein the surgical instrument includes at least one of a reamer or an impactor.
7. The surgical robot of Example 1, wherein the controller is further programmed to progressively guide a tip of the surgical instrument to the point and an axis of the surgical instrument to the axis of the attraction volume.
8. The surgical robot of Example 7, wherein progressively guiding includes providing haptic feedback to guide the tip of the surgical instrument to the point, and subsequently providing haptic feedback to guide the axis of the surgical instrument to the axis of the attraction volume, and wherein the axis of the attraction volume is aligned with the planned trajectory of the surgical instrument for the THA procedure.
9. The surgical robot of Example 8, wherein the controller is configured to operate in multiple modes during the THA procedure, wherein the controller automatically switches between the operating modes in response to detecting the surgical instrument is aligned with the planned trajectory of the surgical instrument for the THA procedure.
10. The surgical robot of Example 9, wherein at least one of the operating modes includes a hinge haptic control mode that limits degrees of freedom of the surgical instrument to permit rotation around the axis thereof.
11. The surgical robot of Example 9, wherein at least one of the multiple operating modes includes a linear guide haptic control mode that limits degrees of freedom of the surgical instrument to permit translation along the axis.
12. The surgical robot of Example 1, wherein the at least one attraction volume has an approximately conical shape.
13. The surgical robot of Example 1, further comprising a navigation system for maintaining alignment of the surgical instrument along the planned trajectory for the THA procedure.
14. The surgical robot of Example 1, wherein the controller is configured to control a reaming portion of the THA procedure by: forcing the surgical instrument into alignment with the planned trajectory for the THA procedure such that a tip of the surgical instrument is coincident with the point, wherein the point defines an initial reaming location; and arresting axial movement of the surgical instrument when the tip reaches a final point that defines a final reaming location, the final reaming location being distal of the initial reaming location, wherein after forcing the surgical instrument into alignment with the planned trajectory, the surgical instrument is free to rotate about the axis or to pivot about the point, and wherein the surgical instrument is free to rotate about the axis or pivot about the point after axial movement has been arrested.
15. The surgical robot of Example 1, further comprising an end effector for holding the surgical instrument, wherein the end effector enables coupling of distinct surgical instruments for the THA procedure.
16. A method of controlling a surgical robot during a total hip arthroplasty (THA) procedure, the method comprising: detecting a position of the surgical instrument in at least one attraction volume corresponding with a planned trajectory of the surgical instrument for the THA procedure, the at least one attraction volume defined in part by a point and an axis extending from the point; and while the surgical instrument is in the at least one attraction volume during the THA procedure, providing assistance to movement of the surgical instrument in a direction toward the at least one attraction volume.
17. The method of Example 16, wherein the at least one attraction volume includes multiple attraction volumes having distinct associated attraction forces, wherein a first attraction volume has a lesser attraction force than a second attraction volume, and wherein the second attraction volume is a sub-volume of the first attraction volume, wherein the point and the axis define a centerline of both the first attraction volume and the second attraction volume, wherein an operator must exert greater force to remove the surgical instrument from the second attraction volume than the first attraction volume, and the method further includes fixing the surgical instrument in alignment with the planned trajectory in at least one operating mode.
18. The method of Example 16, wherein the controller is further programmed to progressively guide a tip of the surgical instrument to the point and an axis of the surgical instrument to the axis of the attraction volume.
19. The method of Example 18, wherein progressively guiding includes providing haptic feedback to guide the tip of the surgical instrument to the point, and subsequently providing haptic feedback to guide the axis of the surgical instrument to the axis of the attraction volume, and wherein the axis of the attraction volume is aligned with the planned trajectory of the surgical instrument for the THA procedure, the method further comprising: automatically switching between operating modes in response to detecting the surgical instrument is aligned with the planned trajectory of the surgical instrument for the THA procedure.
20. The method of Example 19, wherein at least one of the operating modes includes: a hinge haptic control mode that limits degrees of freedom of the surgical instrument to permit rotation around the axis thereof, or a linear guide haptic control mode that limits degrees of freedom of the surgical instrument to permit translation along the axis.
21. The method of Example 16, wherein the controller is configured to control a reaming portion of the THA procedure by: forcing the surgical instrument into alignment with the planned trajectory for the THA procedure such that a tip of the surgical instrument is coincident with the point, wherein the point defines an initial reaming location; and arresting axial movement of the surgical instrument when the tip reaches a final point that defines a final reaming location, the final reaming location being distal of the initial reaming location, wherein after forcing the surgical instrument into alignment with the planned trajectory, the surgical instrument is free to rotate about the axis or to pivot about the point, and wherein the surgical instrument is free to rotate about the axis or pivot about the point after axial movement has been arrested.

## Claims

1. A surgical robot for use in a total hip arthroplasty (THA) procedure, the robot comprising:
- a robot arm for holding a surgical instrument; and
- a controller coupled with the robot arm, the controller programmed to:
- detect a position of the surgical instrument in at least one attraction volume corresponding with a planned trajectory of the surgical instrument for the THA procedure, the at least one attraction volume defined in part by a point and an axis extending from the point; and
- while the surgical instrument is in the at least one attraction volume during the THA procedure, provide assistance to movement of the surgical instrument in a direction toward the at least one attraction volume.

2. The surgical robot of claim 1, wherein the at least one attraction volume includes multiple attraction volumes having distinct associated attraction forces.

3. The surgical robot of claim 2, wherein a first attraction volume has a lesser attraction force than a second attraction volume, and wherein the second attraction volume is a sub-volume of the first attraction volume.

4. The surgical robot of claim 2 or 3, wherein the point and the axis define a centerline of both the first attraction volume and the second attraction volume.

5. The surgical robot of claim 3, wherein an operator must exert greater force to remove the surgical instrument from the second attraction volume than the first attraction volume, and wherein the controller is configured to fix the surgical instrument in alignment with the planned trajectory in at least one operating mode.

6. The surgical robot of any one of the preceding claims, wherein the surgical instrument includes at least one of a reamer or an impactor.

7. The surgical robot of any one of the preceding claims, wherein the controller is further programmed to progressively guide a tip of the surgical instrument to the point and an axis of the surgical instrument to the axis of the attraction volume.

8. The surgical robot of claim 7, wherein progressively guiding includes providing haptic feedback to guide the tip of the surgical instrument to the point, and subsequently providing haptic feedback to guide the axis of the surgical instrument to the axis of the attraction volume, and wherein the axis of the attraction volume is aligned with the planned trajectory of the surgical instrument for the THA procedure.

9. The surgical robot of claim 8, wherein the controller is configured to operate in multiple modes during the THA procedure, wherein the controller automatically switches between the operating modes in response to detecting the surgical instrument is aligned with the planned trajectory of the surgical instrument for the THA procedure.

10. The surgical robot of claim 9, wherein at least one of the operating modes includes a hinge haptic control mode that limits degrees of freedom of the surgical instrument to permit rotation around the axis thereof.

11. The surgical robot of claim 9 or 10, wherein at least one of the multiple operating modes includes a linear guide haptic control mode that limits degrees of freedom of the surgical instrument to permit translation along the axis.

12. The surgical robot of any one of the preceding claims, wherein the at least one attraction volume has an approximately conical shape.

13. The surgical robot of any one of the preceding claims, further comprising a navigation system for maintaining alignment of the surgical instrument along the planned trajectory for the THA procedure.

14. The surgical robot of any one of the preceding claims, wherein the controller is configured to control a reaming portion of the THA procedure by:
- forcing the surgical instrument into alignment with the planned trajectory for the THA procedure such that a tip of the surgical instrument is coincident with the point, wherein the point defines an initial reaming location; and
- arresting axial movement of the surgical instrument when the tip reaches a final point that defines a final reaming location, the final reaming location being distal of the initial reaming location,
- wherein after forcing the surgical instrument into alignment with the planned trajectory, the surgical instrument is free to rotate about the axis or to pivot about the point, and
- wherein the surgical instrument is free to rotate about the axis or pivot about the point after axial movement has been arrested.

15. The surgical robot of any one of the preceding claims, further comprising an end effector for holding the surgical instrument, wherein the end effector enables coupling of distinct surgical instruments for the THA procedure.
